# EUROPEAN PATENT APPLICATION

(11) **EP 4 180 059 A1**
(43) Date of publication of application: **17.05.2023**
(21) Application number: 20939156.4
(22) Date of filing: 29.10.2020
(51) Int. Cl.: A61K 45/00, A61K 38/17, A61P 1/00

(54) **USE OF PHOSPHATIDYLSERINE IN PREPARATION OF DRUG FOR TREATING INFLAMMATORY BOWEL DISEASE**

(30) Priority: 03.06.2020 CN 202010493951
(71) Applicant: Jiangsu Target Biomedical Research Institute Co., Ltd., Changzhou, Jiangsu 213000 (CN)
(72) Inventor: HUA, Zichun, hangzhou, Jiangsu 213000 (CN); WANG, Xinran, hangzhou, Jiangsu 213000 (CN); SONG, Lulu, hangzhou, Jiangsu 213000 (CN); HUO, Lina, hangzhou, Jiangsu 213000 (CN); ZHANG, Xuerui, hangzhou, Jiangsu 213000 (CN)
(74) Representative: Casalonga
(86) International application number: PCT/CN2020/124686
(87) International publication number: WO 2021/243932

(57) **Abstract**

Disclosed is use of phosphatidylserine as a target for treating inflammatory bowel disease. Phosphatidylserine can be used as a marker of inflammatory bowel disease and used for drug screening. Annexin A5 can be traced by phosphatidylserine in the extracellular membrane and indicate the location of lesions and extent of inflammatory bowel disease, and can be used to treat inflammatory bowel disease. Better anti-inflammatory effects can be generated by mutating Annexin A5.

## Description

### Technical Field

The present invention relates to the technical field of biochemistry, and specifically to the use of phosphatidylserine in the preparation of drugs for the treatment of inflammatory bowel disease.

### Background Art

Annexin A5 has a variety of functions in living organisms, such as antitumor and anticoagulation. Annexin A5 can interfere with the recruitment and activation of monocytes at the site of inflammatory lesions, thereby reducing plaque inflammation at the lesion site.

Inflammatory bowel disease (IBD) is a chronic and recurring disease, including Crohn's disease (CD) and ulcerative colitis (UC), which often presents with abdominal pain, diarrhea, blood in the stool, anemia and weight loss, and greatly affects the quality of life of patients. The pathogenesis of IBD is complex, and there are many influencing factors in its development. Therefore, the pathogenesis and treatment of IBD have been the focus of research. When IBD occurs, the intestinal mucosa is damaged, the intestinal villous structure is broken, inflammatory cell infiltration increases, and the level of inflammatory cytokine secretion increases.

Based on the anti-inflammatory function of Annexin A5, the present invention focuses on the expression level of Annexin A5 in colonic tissues. By examining colonic tissues from clinical patients and TNBS-induced colitis model mice, it was found that the expression of Annexin A5 in the inflammatory tissues was significantly downregulated.

Inflammatory bowel disease (IBD) is a chronic and recurring disease that manifests clinically with recurrent episodes of abdominal pain, diarrhea, and mucopurulent bloody stools, with long treatment cycles that severely affect patients' quality of life. The current treatment of IBD is mainly to control inflammation and regulate immune disorders to control disease occurrence and ease the symptoms. With the in-depth study of the pathogenesis of IBD, some new pharmacological agents for the treatment of IBD have been applied in the clinic, such as Infliximab, a TNF-α antibody agent, which is effective as an antibody drug, but also has non-negligible side effects, such as autoimmune diseases and hematological disorders. Therefore, the development of effective therapeutic drugs for IBD has been in progress.

Annexin A5, a member of Annexin family ofCa2+-dependent phospholipid-binding proteins, has been widely used as a diagnostic tool for detecting apoptosis in in vivo and in vitro studies because of its strong affinity for PS. In addition to detecting apoptotic cells, it has been shown to have anticoagulant, antitumor and anti-inflammatory effects. For example, treatment with Annexin A5 injection can inhibit the recruitment and activation of monocytes to the site of inflammatory lesions and reduce plaque inflammation in advanced lesions in apoE-/- mice. Based on the anti-inflammatory effect of Annexin A5, the present invention focused on Annexin A5 expression in clinical samples from CD and UC patients. Compared to normal colonic mucosal tissues, the present invention has surprisingly found significantly lower Annexin A5 expression levels in inflammatory colonic mucosal tissues, which suggests a possible important protective role of Annexin A5 in intestinal tissues.

Currently, there is no use of phosphatidylserine in the preparation of drugs for the treatment of inflammatory bowel disease.

### Summary of the Invention

The purpose of the present invention is to provide a use of phosphatidylserine in the preparation of drugs for the treatment of inflammatory bowel disease.

The purpose of the present invention is achieved by the following technical solution: a use of phosphatidylserine in preparation of a drug for treatment of inflammatory bowel disease, wherein for the inflammatory bowel disease, phosphatidylserine serves as a target of the drug.

Further, in the inflammatory bowel disease, phosphatidylserine of vascular endothelial cells is flipped from the intracellular membrane to the extracellular membrane, and the externalized phosphatidylserine serves as a marker of inflammatory bowel disease.

Further, marked Annexin A5 is capable of being traced by phosphatidylserine in the extracellular membrane and indicating location of lesions and extent of the inflammatory bowel disease.

Further, the drug is screened by using phosphatidylserine as the target for the inflammatory bowel disease to obtain proteins and compounds having a high affinity to phosphatidylserine.

Further, the drug exerts anti-inflammatory effects by binding to phosphatidylserine to inhibit inflammatory cell infiltration and block recruitment of colonic immune cells.

Further, Annexin A5 exerts anti-inflammatory effects by binding to phosphatidylserine.

A method of modifying a drug that uses phosphatidylserine as a target for inflammatory bowel disease, wherein Annexin A5 is mutated to enhance its binding ability to phosphatidylserine to improve anti-inflammatory effects thereof.

Beneficial effects: The present invention has confirmed Annexin A5 has significant efficacy in the treatment of TNBS-induced colitis and, for the first time, has found that it can exert anti-inflammatory effects by being enriched in the capillaries of intestinal mucosa via PS-specific binding to inhibit adhesion and infiltration of inflammatory cells. This can be an emerging potential therapeutic strategy for inflammatory bowel disease (IBD).

### Brief Description of the Drawings

The present invention will be further described below with reference to the accompanying drawings, in which:
Figure 1 is a schematic representation of Annexin A5 expression in human colon tissues by Western blot assay of the present invention, wherein FIG. 1A shows the Annexin A5 protein expression level of the present invention; FIG. 1B shows the quantitative analysis of the expression level of the present invention; in FIG. 1, 1-4, normal tissues adjacent to the lesion area of clinical samples from four different patients and the lesion area of CD patients; 5, 6 , normal colon tissue; 7, lesion area of CD patients; 8, 9, lesion area of UC patients.
Figure 2 is a schematic representation of the Western blot assay of the present invention for Annexin A5 expression in mouse colon tissue, wherein FIG. 2A shows the normal mouse colon morphology and model mouse colon morphology of the present invention; FIG. 2B shows the expression of Annexin A5 in colon tissues of the present invention; FIG. 2C shows quantitative analysis of Annexin A5 expression levels. 1-5, normal tissues from healthy mice; 6-10, inflammatory tissues from mice with TNBS-induced colitis.
Figure 3 shows a flow chart of the operation of the **adoptive** experiment of the present invention.

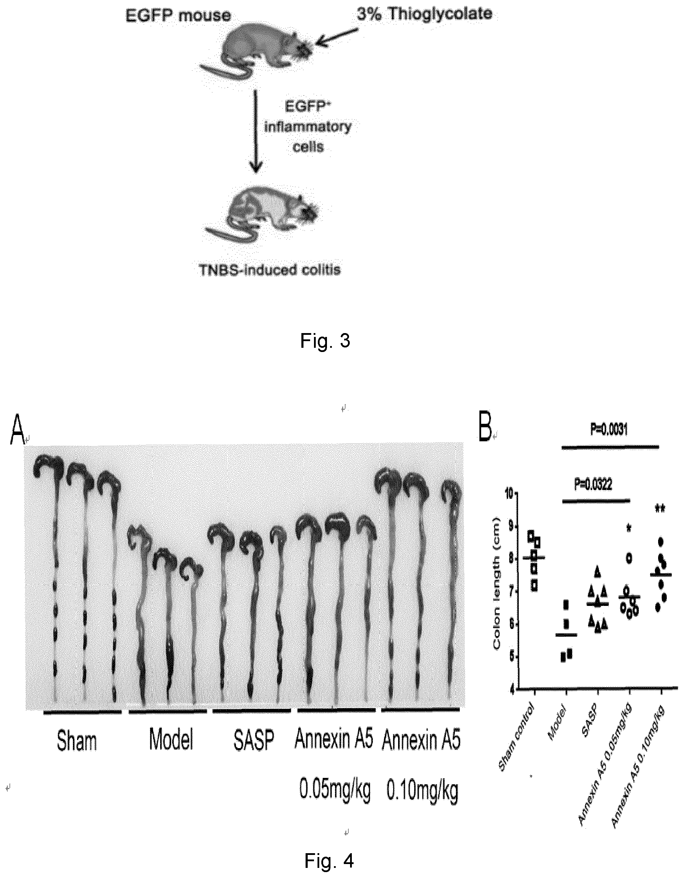

Figure 4 is the schematic representation of the colon morphology and length measurement of the TNBS-induced colitis model of the present invention, wherein Figure 4A shows the observations of colon morphology of the present invention; Figure 4B shows the statistics of colon length measurements of the present invention, wherein sham control represents normal mouse colon; Model represents TNBS enema only; SASP represents SASP treatment group, all positive controls; Annexin A5 0.05 mg/kg represents being administered at 0.05 mg/kg dose; Annexin A5 0.10 mg/kg represents being administered at a dose of 0.10 mg/kg, and data statistics are mean ± SEM.
Figure 5 shows the trend of body weight change and DAI score over time for model mice of the present invention, wherein Figure 5A shows the trend of body weight change over time for each group of the present invention, wherein mice were weighed every morning after modeling; Figure 5B shows the change of DAI score over time for each group of the present invention, wherein the data were calculated by DAI calculation formula based on the daily weight change, fecal hardness and occult blood, with the data statistics being mean ± SEM.
Figure 6 shows the H&E staining results and scores of pathological tissue sections after TNBS-induced colitis of the present invention, wherein Figure 6A shows the H&E staining results of histopathological sections of the present invention; Figure 6B shows the histopathological score of each group of the present invention. The extent of intestinal wall damage, lesions and crypt damage was investigated and the calculation was based on the histopathological scoring formula. The arrows show the areas of intestinal mucosa and crypt damage.
Figure 7 is the diagram of serum cytokine level assay for each group of the present invention.
Figure 8 is a schematic representation of the flow cytometry analysis of colonic mucosal leukocytes of the present invention.
Figure 9 shows the immunofluorescent diagram of leukocytes in colonic inflammatory tissue of the present invention.
Figure 10 is a schematic representation of the infiltration of EGFP+ inflammatory cells at the site of colonic inflammation of the present invention.
Figure 11 shows the infiltration of the adoptive EGFP+ inflammatory cells in mouse colonic tissue of the present invention.
Figure 12 shows a schematic representation of the inhibitory effect of Annexin A5 on the adhesion of THP-1 to HUVEC of the present invention.
Figure 13 shows the in vivo distribution of Annexin A5-TagRFP in tissues of the mice of the present invention.

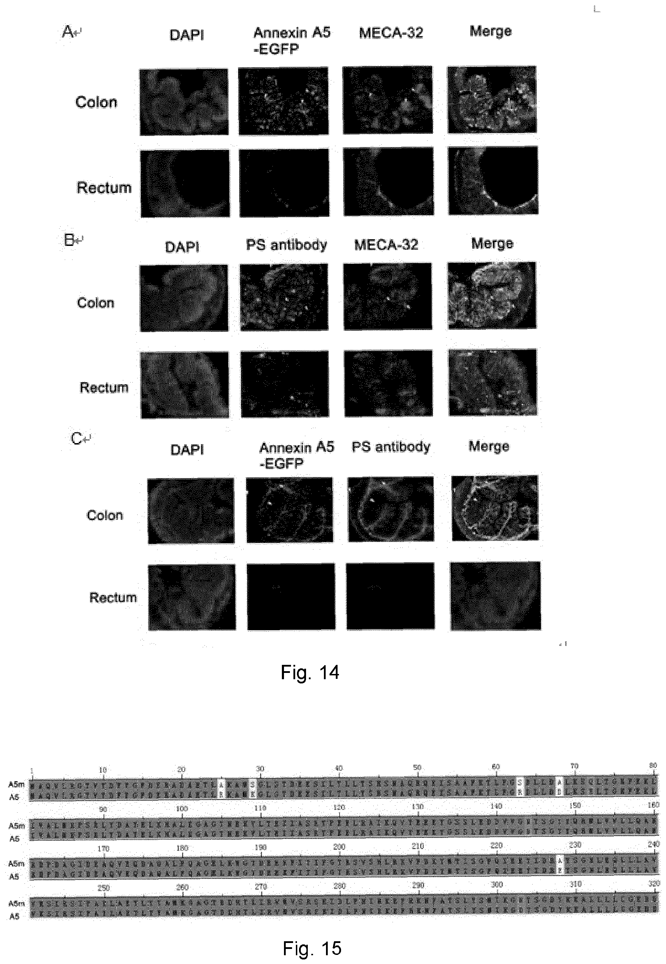

Figure 14 shows the immunofluorescence detection of colon and ileal tissues of the present invention, wherein Figure 14A shows co-localization of Annexin A5-EGFP and MECA-32-labeled capillaries in the intestinal mucosa of the present invention, with the blue color representing the nucleus, the green fluorescence representing AnnexinA5-EGFP protein enriched in the colon, the red color representing capillaries, and the yellow color representing green-red co-localization; Figure 14B shows co-localization of PS and MECA-32-labeled capillaries in the intestinal mucosa of the present invention, with the green color representing externalized PS; Figure 14C shows co-localization of Annexin A5-EGFP and PS in intestinal mucosa of the present invention, with the green fluorescence representing AnnexinA5-EGFP, and the red color representing PS.
Figure 15 shows the mutation of the binding sites of Annexin A5 to PS of the present invention.
Figure 16 shows a linear model of the three-dimensional structure of Annexin A5/Annexin A5m and the results of docking with PS of the present invention.
Figure 17 shows the purification results of Annexin A5m-related protein detected by SDA-PAGE of the present invention.
Figure 18 shows the secondary structures of Annexin A5m and Annexin A5 detected by circular dichroism of the present invention.
Figure 19 shows the binding power analysis (MST) of Annexin A5-EGFP/Annexin A5m-EGFP with PS of the present invention.
Figure 20 shows the induced apoptosis of A549 cells detected by Annexin A5-EGFP/Annexin A5m-EGFP of the present invention.
Figure 21 shows the distribution of Annexin A5-TagRFP/Annexin A5m-TagRFP in vivo and in organs of mice of the present invention, wherein Figure 21A shows the distribution of Annexin A5-TagRFP/Annexin A5m-TagRFP in living mice of the present invention; Figure 21B shows the distribution of Annexin A5-TagRFP/Annexin A5m-TagRFP in organs of the present invention.
Figure 22 shows the localization of Annexin A5/A5m-EGFP in colonic tissue of the present invention.
Figure 23 shows the appearance and length of the colon section after TNBS-induced colitis of the present invention, wherein herein Figure 23A shows the appearance of colon of each group of mice of the present invention, with Sham representing normal mice, Model group representing perfused with TNBS only, A5/A5m representing administered groups respectively; Figure 23B shows the statistics of colon length of each group of the present invention.
Figure 24 shows the trend of body weight change and DAI score of mice of the present invention, wherein Figure 24A shows the trend of body weight change over time of each group of the present invention; Figure 24B shows the trend of DAI score changeover time of each group of the present invention.
Figure 25 shows the H&E staining and pathological score of the colon tissue of TNBS-induced colitis model mice of the present invention.
Figure 26 is a schematic representation of flow cytometry analysis of colonic mucosal leukocytes of the present invention.

### Detailed Description of the Invention

The invention is described in further detail by the following embodiments, but it should be noted that the scope of the invention is not limited in any way by these embodiments.

### Embodiment 1

A use of phosphatidylserine in preparation of a drug for treatment of inflammatory bowel disease of the present invention, wherein for the inflammatory bowel disease, phosphatidylserine serves as a target of the drug.

In the inflammatory bowel disease, phosphatidylserine of vascular endothelial cells is flipped from the intracellular membrane to the extracellular membrane, and the flipped phosphatidylserine serves as a marker of inflammatory bowel disease.

Marked Annexin A5 is capable of being traced by phosphatidylserine in the extracellular membrane and indicating location of lesions and extent of the inflammatory bowel disease.

The drug is screened by using phosphatidylserine as the target for the inflammatory bowel disease to obtain proteins and compounds having a high affinity to phosphatidylserine.

The drug exerts anti-inflammatory effects by binding to phosphatidylserine to inhibit inflammatory cell infiltration and block recruitment of colonic immune cells. Annexin A5 exerts anti-inflammatory effects by binding to phosphatidylserine.

A method of modifying a drug that uses phosphatidylserine as a target for inflammatory bowel disease of the present invention, wherein Annexin A5 is mutated to enhance its binding ability to phosphatidylserine to improve anti-inflammatory effects thereof.

The present invention constructed the most commonly used IBD disease model in mice - TNBS chemically induced colitis. For the TNBS disease model, its histological characteristics, clinical manifestations, pathogenesis site and inflammatory factors are very similar to IBD. The model mice showed clinical signs such as diarrhea, mucus and blood stools, and gradual weight loss, indicating that the enteritis model was successfully constructed. Consistent with the clinical data, the expression level of Annexin A5 in colonic tissues was significantly downregulated with the development of enteritis in mice, which further verifies the correlation between the decrease of Annexin A5 and the development of enteritis. What is the link between Annexin A5 expression level and the development of enteritis, and can Annexin A5 treat IBD?

The present invention firstly explored the effect and mechanism of action of Annexin A5 in the treatment of colitis. First, the present invention tested the efficacy of Annexin A5 administration on the TNBS colitis model, and evaluated the colitis condition from various indexes such as overall status of the mice, weight change, and extent of intestinal mucosal damage. The results showed that the Annexin A5 treatment group had significant anti-inflammatory effects and the degree of colitis was greatly alleviated. By further immunofluorescence and flow analysis of Annexin A5's anti-inflammatory mechanism of action, the present invention revealed that Annexin A5 was able to significantly inhibit the infiltration of inflammatory cells and the production of inflammatory factors, thereby exerting an anti-inflammatory effect. The results were verified by EGFP-labeled inflammatory cell adoptive assay, which clearly demonstrated that Annexin A5 could significantly inhibit the adhesion and infiltration of inflammatory cells.

Subsequently, studies on the therapeutic mechanism of Annexin A5 were conducted. The present invention successfully expressed and purified Annexin A5-TagRFP recombinant protein labeled with red fluorescent, which was injected intravenously for imaging of live mice. It was found that Annexin A5 was significantly enriched in intestinal tissues. For further confirmation, the present invention performed fluorescence analysis of intestinal tissues in sections and obtained a surprising result: there was a large scale of PS externalization in the capillaries in the intestinal mucosa, and Annexin A5 co-localized well with the externalized PS site, suggesting that Annexin A5 enrichment and binding to intestinal tissues was achieved by PS-specific binding. To further confirm this, a mutant A5m of Annexin A5 was designed and expressed, which retained the secondary protein structure of wild-type Annexin A5 and only lacked the PS binding ability. A series of subsequent experiments demonstrated that when Annexin A5 lacked PS binding ability, Annexin A5m was not able to be enriched in the colon, thereby failing to inhibit inflammatory cell infiltration and alleviate the symptoms of colitis, and thus had no efficacy at all. These results sufficiently demonstrated that the therapeutic effect of Annexin A5 was PS-dependent and can be applied to a therapeutic strategy that a targeted drug is delivered dependent on PS externalization, which is promising in development of anti-inflammatory drugs for treatment of IBD.

### Example 1

### 1.1 Experimental materials

### 1.1.1 Samples

Colon samples of clinical patients were collected from Jiangsu Provincial People's Hospital; C57BL/6 mice were obtained from the Model Animal Institute of Nanjing University.

### 1.1.2 Antibodies and reagents

Annexin A5 antibody was obtained from CST; protein molecular weight standard Protein Marker was obtained from Shanghai Invitrogen; BCA kit was obtained from Shanghai Beyotime; TNBS and Ammonium persulfate (AP) were obtained from Sigma; PVDF membrane was obtained from Millipore; Acrylamide and TEMED were obtained from Bio-Rad Corporation, United States; DAPI staining solution was obtained from Invitrogen Corporation; Glycine and Tris were obtained from Nanjing SunShine Biotechnology; other analytical reagents such as anhydrous ethanol and methanol were obtained from Nanjing Chemical Reagent Factory.

### 1.1.3 Instruments

Tissue homogenizer; Full-wavelength multifunctional enzyme standardizer, model Safire, obtained from Tecan, Switzerland; Chemiluminescence imaging system obtained from Shanghai Tanon.

### 1.1.4 Solution

10% SDS solution: 10 g SDS was dissolved in 100 mL ddH₂O, placed in 50°C environment for dissolution, and then stored at room temperature.

10% ammonium persulfate (AP) solution: mass to volume ratio, 0.1 g AP was added to 1.0 mL ddH₂ O, dissolved and then stored at 4°C.

1.5 M Tris-HCl (pH 8.8): Tris 18.17 g was dissolved in about 80 mL of ddH₂O. After dissolved, the pH was adjusted to 8.8 with concentrated hydrochloric acid, the volume was fixed to 100 mL and then stored at room temperature.

1.0 M Tris-HCl (pH 6.8): 114 g of Tris was dissolved in about 80 mL of ddH₂O.The pH was adjusted after dissolution and the volume of the solution was fixed to 100 mL and then stored at room temperature.

5×SDS-PAGE electrophoresis buffer: 94 g glycine, 75.5 g Tris and 25 g SDS were dissolved in about 800 mL ddH₂ O, the volume was fixed to 1 L, and store at room temperature.

5× SDS-PAGE loading buffer: 250 mM Tris-HCl (pH 6.8), 10% (w/v) SDS, 0.5% (w/v) BPB (bromophenol blue) and 50% (w/v) glycerol were store at room temperature, 5% mercaptoethanol was added before use.

10× wet transfer buffer: 144 g glycine and 33.3 g Tris were dissolved in ddH₂O and the volume was fixed to 1 L, diluted to 1× before use, and methanol was added, with the ratio being: 100 mL 10× wet transfer buffer + 700 mL ddH₂ O + 200 mL methanol.

10×TBS buffer: Tris 24.2 g and NaCl 80.0 g were weighed, the pH was adjusted to 7.6 with diluted hydrochloric acid, volume fixed to 1 L, dissolved fully and then stored at room temperature.

1×TBST buffer: 100 mL 10×TBS buffer + 900 mL ddH₂ O+ 1 mL Tween-20, dissolved fully and then store at room temperature.

(10) The 12 % SDS-PAGE protein gel formulation system is shown in Table 1 below.

**Table 1-1 Formulation of 5% Concentrated Gel**

| Components | Vol. (mL) for 3mL Gel |
|---|---|
| ddH20 | 2.1 |
| 30% Acrylamide | 0.5 |
| 1M Tris-HCL (pH 6. 8) | 0. 38 |
| 10% AP | 0. 03 |
| 10%SDS | 0.03 |
| TEMED | 0.03 |

**Table 1-2 Formulation of 12% Separation Gel**

| Components | Vol. (mL) for 10 mL Gel |
|---|---|
| ddH20 | 3.3 |
| 30% Acrylamide | 4.0 |
| 1.5M Tris-HCL (pH 8.8) | 2.5 |
| 10% AP | 0.1 |
| 10%SDS | 0.1 |
| TEMED | 0.004 |

### 1.2 Experimental methods

### 1.2.1 Mice with TNBS-induced colitis

The previous method was slightly improved by selecting a number of 16-18 g mice and placing them in metabolic cages for 24 h with fasting.

Drug formulation: 50% anhydrous ethanol + 48% of 5% TNBS mother liquor + 2% ddH₂O.

After the mice were anesthetized by ether inhalation, 0.1 mL of the formulated drug was taken into a syringe and connected to a gavage needle (with 1.2 mm inner diameter), the mice were secured with one hand, and the catheter was rotated into the colon about 5-6 centimeters from the anus with the other hand. For the TNBS model group, a final concentration of 0.1 MI TNBS was perfused slowly. For the control group, equivalent amount of PBS was perfused. During the perfusion, the head should be higher than the tail to preventing the liquid from overflow. After all the liquid was pushed into the colon, the gavage needle was slowly withdrawn and the mice were hung upside down for 1 min after perfusion.

Then natural waking; routine feeding.

Body weights were recorded daily and the mice were observed for stool status, eating situation and mental state.

### 1.2.2 Determination of protein concentration by BCA method

Standard dilution: the diluents was the same as the sample to be tested. BCA working solution was prepared according to Table 2.

**Table 2**

| Tube # | Diluent Vol. | Amount of BSA Standard (µL) | Concentration of BSA Standard (µL) |
|---|---|---|---|
| A | 0 | 100 | 2 |
| B | 200 | 200 | 1 |
| C | 200 | 200 | 0.5 |
| D | 200 | 200 | 0.25 |
| E | 200 | 200 | 0.125 |
| F | 200 | 200 | 0.0625 |
| G | 200 | 0 | 0 |

The total amount of samples and the total number of replicate wells were calculated, with 200µL of working solution for each well. The ratio of 50: 1 (A: B) was used to mix A and B solutions. Note that an extra 1/10 of the amount was used to prevent shortages caused by errors.

### BCA reaction

The samples were diluted at appropriate ratios, 25 µL of the sample to be tested and 25 µL of the standard were added respectively to a 96-well plate, and 200 µL of BCA working solution was added, shaken and mixed gently, and placed at 37°C for 20-30 minutes.

### Testing

The absorbance values were measured at 562 nm using an enzyme standardizer within 10 minutes after the end of the reaction and a standard curve was drafted. The concentration of the protein to be measured was calculated.

### 1.2.3 Detection of Annexin A5 expression in colonic tissues

Protein sample preparation: Colon tissues were rinsed in PBS to remove blood, then cut up and added with an appropriate amount of Western blot and IP lysis solution (containing protease inhibitor), broken up by tissue homogenizer and filtered by 100m cell sieve, and the filtrate was collected into a new EP tube. Homogenized 10 s each time, with an interval of 20 s, for 3 minutes. Note low temperature for all operations.

After 40 minutes on ice, centrifuged at 12000 rpm for 10 minutes at 4°C and the supernatant was taken.

The concentration of the protein solution was measured by BCA method. The samples were added to 5× loading buffer, boiling water bath for 10 minutes, and then cooled and stored at -20°C.

Electrophoresis: 12% SDS-PAGE gel was prepared. According to the sample concentration, the loading volume was calculated based on a loading amount of 30 µg. A voltage of 80 V was used for concentration and 120 V was used for separation.

Wet transfer: The transfer buffer-soaked filter paper, gum, PVDF membrane (pre-soaked in methanol for 1 minute), and filter paper were placed in sequence on the transfer clamp from the cathode (black plate) to the anode (white plate), and the air bubbles were removed; the transfer clamp was tightened, and then placed into a transfer tank while maintaining the low temperature by placing a bio-ice pack therein. The membrane transfer was performed at a constant flow of 300 mA for 90 minutes.

Blocking: The transferred film was put into 50 mL of 5 % BSA or skim milk for blocking at room temperature for 1 h or overnight at 4 °C.

Primary antibody coating: After closure, the primary antibody was diluted with 5% skim milk (1:1000) and the membrane was incubated in the primary antibody for 6 h at room temperature or overnight at 4°C.

Excess primary antibody washing off: The membrane was washed 3 times with 1 × TBST, 5 minutes for each time.

Secondary antibody coating: the secondary antibody was diluted in 5 % skim milk; coated with the secondary antibody, and incubated for 30 minutes at room temperature.

Excess secondary antibody washing off: The membrane was washed 3 times with 1 × TBST, 5 minutes for each time.

Luminescence detection: After 30 s of dropwise addition of luminescent solution, the membrane was put into a chemiluminescence instrument and photographed to obtain the target protein bands.

### 1.3 Experimental results and analysis

### 1.3.1 Clinical data on human colitis show that Annexin A5 expression is negatively correlated with the development of colitis

Biopsy samples of clinical patients with colitis were collected from hospitals were used for Annexin A5 expression analysis.

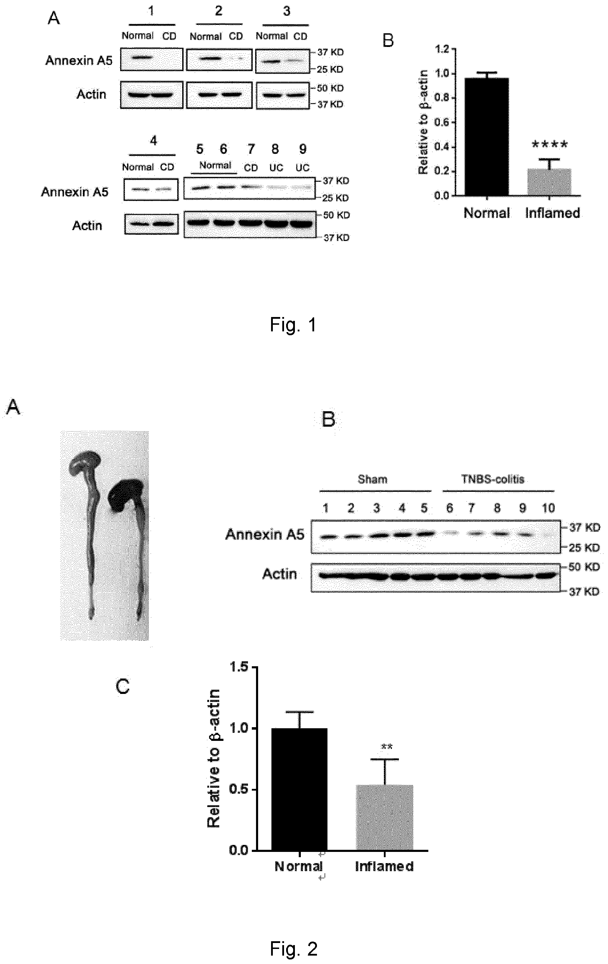

Western blot results show in Figure 1 of the present invention that Annexin A5 expression in human colon tissues was downregulated in the enteritis group including CD and UC compared to normal tissues as detected by Western blot (Figure 1A). Quantitative analysis showed that Annexin A5 expression was significantly downregulated (p<0.001) (Figure 1B). Annexin A5 protein expression levels; B. Quantitative analysis of expression levels. 1-4, normal tissues adjacent to the lesion area in clinical samples from four different patients and the lesion area in patients with CD; 5 and 6, normal colon tissues; 7, lesion area in patients with CD; 8 and 9, lesion areas of UC patients.

### 1.3.2 Mouse TNBS-induced colitis model verified decreased Annexin A5 expression levels in induced enteritis

The finding of significant downregulation of Annexin A5 expression levels in colonic mucosal tissues of clinical patients under inflammatory states suggested that Annexin A5 levels may be correlated with the occurrence of enteritis. For this reason, the present invention was validated using TNBS to induce colitis in mice. After modeling, mice showed diarrhea, bloody stools, weight loss, and a certain degree of redness, swelling and shortening of the colon, indicating that the colitis model was successfully constructed (Figure 2A). Mouse colonic tissues of successfully TNBS-induced colitis were taken, homogenized and then the protein was extracted for analysis of Annexin A5 expression. Western blot results showed that the expression of Annexin A5 was significantly downregulated in colitis compared with normal tissues (Figure 2B, 2C). Figure 2 is a schematic representation of Annexin A5 expression in mouse colonic tissues as detected by Western blot of the present invention; Figure 2A shows the colonic morphology of normal and colitis mice; Figure 2B shows expression of Annexin A5 in colonic tissues; Figure 2C shows quantitative analysis of Annexin A5 expression levels. 1-5, normal tissues from healthy mice; 6-10, inflammatory tissues from mice with TNBS-induced colitis.

### 1.4 Summary and Discussion

The results of the colitis mice and the clinical samples were consistent. With the development of enteritis in mice, the expression level of Annexin A5 in colonic tissues was significantly down-regulated, further verifying the correlation between the reduction of Annexin A5 and the development of enteritis.

### Example 2

Annexin A5 was found to be expressed at decreased levels during the development of enteritis, so is it possible to protect the intestine and alleviate enteritis by supplementing with exogenous Annexin A5 recombinant protein? The present invention expresses and purifies the recombinant protein Annexin A5 for use in a mouse model of enteritis treatment. An appropriate animal model is very important in the research of occurrence and development of a disease and discovery of drugs. TNBS induction is a model often used in laboratory studies of inflammatory bowel disease (IBD), and TNBS-induced colitis is mainly mediated by Th1-type cells, similar to the pathogenesis of human CD. Therefore, it is used as a common animal model for studying CD and can be used for the therapeutic drug development to explore the therapeutic effect of the drug. TNBS-induced colitis is characterized by apparent weight loss, mental lethargy, inflammation throughout the intestine, with thickening of the intestinal wall and damage to the intestinal villi as the major characteristics, increased inflammatory cell infiltration, and increased secretion of associated inflammatory cytokines. With respect to the above-referenced enteritis disease indicators, the present invention conducted relevant studies.

### 2.1 Experimental materials

### 2.1.1 Animals

16-18g C57BL/6 mice were obtained from the Model Animal Research Center of Nanjing University; EGFP mice were raised for this experiment.

### 2.1.2 Reagents

Paraformaldehyde (PFA) was obtained from Sigma; 10×PBS was obtained from Nanjing SunShine Biotechnology; RPMI 1640; suspension array; Percoll master liquor; thioglycolate was obtained from Beijing Solarbio; BSA; HEPES; Glycine; NaHCO3; DTE (dithioerythritol); RPMI 1640 and fetal bovine serum (FBS) was obtained from Thermo; occult blood kit; BCA kit was obtained from Beyotime; primers and gene synthesis were obtained from Nanjing GenScript; DAPI staining solution was obtained from Invitrogen Corporation; Taipan Blue; CellTracker Green CMFDA was obtained from Invitrogen; Hoechst was obtained from Invitrogen..

### 2.1.3 Antibodies and proteins

Annexin A5 (purified in our laboratory); Annexin A5-EGFP protein (stored in our laboratory); Phosphatidylserine (PS) antibody was obtained from R&D; MECA-32 antibody was obtained from BD Biosciences; CD11b antibody was obtained from BD Biosciences; AlexaFluor-conjugated secondary antibody was obtained from Invitrogen Corporation; Protein Marker was obtained from Shanghai Invitrogen; TNF-α was obtained from Invitrogen.

### 2.1.4 Instruments and consumables

Flow cytometer was obtained from BD; pH meter was obtained from Mettler Toledo, Switzerland; shaker was obtained from JiangsuKylin-Bell; automatic cell counter was obtained from Invitrogen; autoclave was obtained from Nanjing EPED; fluorescence microscope was obtained from Carl Zeiss AG, Germany, model Zeiss AX10; automatic frozen section machine CM1950 was obtained from Leica; Gavage needles were obtained from Xinhua Medical Devices; scales; forceps; 10 mL and 20 mL syringes; 50 mL conical centrifuge tubes; 50 mL conical flasks.

### 2.1.5 Solution

(1) 10× HEPES-sodium bicarbonate buffer: 23.8 g HEPES (100 mM), 21 g sodium bicarbonate (250 mM) were added with water to 1 L, pH was adjusted to 7.2 with HCl. Stored at room temperature for no more than 1 month.
(2) CMF solution: 100 mL 10×HBSS (free of calcium and magnesium), 100 mL 10×HEPES-sodium bicarbonate buffer, and 20 mL FBS were added with water to 1 L, filtered and sterilized. Stored 4°C up to two weeks. Only 800 mL needed for 4 samples.
(3) CMF/FBS/DTE: 8 mL FBS was added to 92 mL CMF, then added with 15.4 mg DTE (dithioerythritol) (ready-to-use) , only 200 mL needed for 4 samples.
(4) 1×Percoll stock solution (ready-to-use): 90 mL Percoll added to 10 mL 10×PBS.
(5) 44% Percoll solution: 44 mL of 1× Percoll stock solution was added to 56 mL of RPMI 1640.
(6) 67% Percoll solution: 67 mL of 1×Percoll stock solution was added to 33 mL of RPMI 1640. pH adjusted to 7.2 with HCl and placed on ice until use. All solutions should be kept at 4°C or in an ice bath when used.
(7) 3% thioglycolate solution: 0.3 g thioglycolate dissolved in 10 mL ddH₂O, sterilized and ready to use.

### 2.2 Experimental methods

### 2.2.1 Assessment of the severity of enteritis in mice

The severity of enteritis in mice was assessed primarily based on the disease activity index (DAI), including weight loss, fecal hardness, and bloody stools. Each item was scored from 0 to 4, with a total of 12 points, and then divided by 3 to obtain the average value. The specific criteria are shown in Table 3 below.

**Table 3**

| Disease Activity Index (DAI) Scoring System | | | |
|---|---|---|---|
| Score | Wt. Loss | Fecal state | Fecal occult blood or Perianal state |
| **0** | **/** | Normal | Normal |
| **1** | **1-5%** | Loose | |
| **2** | **5-10%** | | |
| **3** | **10-20%** | | Occult Blood (+) |
| **4** | **>20%** | Diarrhea | Massive bleeding |

The body weights of the mice in each group were weighed and recorded daily, and the percentages of body weight loss were calculated. The formula was: weight loss (%) on day n = (weight on day n-1 - weight on day n)/weight on day n-1, and weight gain was recorded as 0. The status of mice in each group and symptoms of mucus, fecal blood and diarrhea were observed, and the number of cases of their occurrence was recorded and scored. The assessment was performed with reference to the criteria of Murano [6] et al., with DAI = (weight loss + fecal state + fecal blood)/3.

### 2.2.2 H&E staining

Hematoxylin (H), a basic dye, can stain and the ribosomes in the nucleus blue-purple. Eosin (E), an acidic dye, can stain the cytoplasm red or light red.
(1) A section of colonic inflammation was cliped and put into PFA fixative to denature and coagulate the tissue proteins.
(2) Gradually dehydrated by alcohol of gradient concentrations → xylene immersion wax embedding.
(3) The tissue block was immersed in paraffin wax for embedding, and then cooled.
(4) The wax block was fixed and cut into 5 µm slices; smoothed in hot water and then put on a glass slide and dried.
(5) Staining: hematoxylin solution for a few minutes →color separation in acid water and ammonia for a few seconds → water rinse for 1 h → distilled water for a moment → 70% alcohol dehydration for 10 minutes → 90% alcohol dehydration for 10 minutes → alcohol eosin staining solution for 3 minutes.

Using gum to seal the piece.

### 2.2.3 Histopathological section observation

### (1) H&E staining of pathological tissue sections and observation

After mice were executed, colonic specimens were obtained and fixed with 10% PFA, and paraffin sections were prepared by common practice, stained with H&E, and observed under the microscope.

### (2) Histological scoring of colonic pathology sections

The degree of histological inflammation was scored by a physician who was fully blinded with the experimental design and operation. The criteria are listed in the following table.
(a) Severity of inflammation of the intestinal wall, with 1, 2, and 3 points representing mild, moderate, and severe inflammation, respectively.
(b) Severity of lesion, with 1, 2, and 3 points representing lesion located in the mucosal layer, in the mucosa and mucosal layer, or transmural breakdown, respectively.
(c) The degree of recessus damage was recorded as 1, 2, 3, and 4 points representing, respectively, 1/3 recessus damage (mild), 2/3 recessus damage (moderate), absence of recessus but intact surface epithelium, and complete absence of recessus surface epithelium. Details are shown in Table 4 below.

**Table 4**

| Histological Scoring of Colon Tissue | | |
|---|---|---|
| Items | Scores | Features |
| Inflammation | 0 | / |
| | 1 | Mild |
| | 2 | Moderate |
| | 3 | Severe |
| Degree of Recessus Damage | 0 | / |
| | 1 | 1/3 Base Damage |
| | 2 | 2/3 Base |
| | 3 | Intact Surface Epithelium Only |
| | 4 | Tot. Recessus and Epithelium Dam. |
| Damage Zone | 0 | / |
| | 1 | Mocusa |
| | 2 | Mocusa and Submocusa |
| | 3 | Transmural Damage |
| Damage Area | 0 | 1-25% |
| | 1 | 26-50% |
| | 2 | 51-75% |
| | 3 | 76-100% |

The scores of the above 3 indices were multiplied by the proportion of each lesion area to obtain the final score. The absence of significant lesions was recorded as 0. All scores were accumulated to give a total score, and the mean score (*X̅* ± SD) of each animal in each group was calculated and t-test between groups was performed.

### 2.2.4 Measurement of fecal occult blood in mice

Principle: Hemoglobin contains ferrous hemoglobin, which has peroxidase activity and can catalyze the release of O₂ from H₂O₂ to oxidize o-toluidine into o-toluene azoic and show blue color. The experimental steps were as follows:
(1) Picking a small amount of fresh feces with the tip of a gun and apply it onto a white porcelain plate.
(2) Adding 2 to 3 drops of 0.15 g/L o-toluidine and 2 to 3 drops of H₂O₂ to the feces.
(3) Observing the results immediately.

Assessment of experimental results: (-) Color did not appear after 2 minutes, (+) change from light blue to blue after 10 s, (+ +) change from light blue-brown to blue-brown after adding reagents, (+ + + +) change to blue-black brown immediately.

### 2.2.5 Immunofluorescence assay

For the in vivo localization of Annexin A5 and externalized PS, Annexin A5-EGFP was injected intravenously and colon sections were prepared 20 minutes later, closed with 3% BSA for 1 h, incubated with primary antibody for 2h, followed by incubation with appropriate AlexaFluor-conjugated secondary antibody for 1 h. Counterstain with DAPI at room temperature. The primary antibody used was against CD11b (1:100) and MECA-32 (1:100). Images were observed under a fluorescent microscope.

### 2.2.6 Intestinal epithelial lymphocyte extraction

(1) Executing mice, with a midline abdominal incision, and stretching the skin tightly.
(2) Picking up the tip of the intestine with forceps, slowly dragging the colon out of the abdominal cavity, cutting the lower end at the appendix, and dragging the colon out to the anus and cutting it off.
(3) Filling a 20 mL syringe with 4°C CMF solution, picking up the colon with forceps, placing it in a large beaker, inserting an 18- G or 20-G needle into the intestinal lumen, and slowly applying pressure (5 mL per minute) to the syringe barrel. If leakage or blockage occurs during flushing, move the forceps to the problem area, reinsert the syringe, and continue to instill CMF. Flush the entire intestine with 40 mL of 4°C CMF.
(4) Placing the intestinal tissue on CMF-moistened filter paper and using two forceps to remove the remaining fat and connecting tissue; placing the forceps horizontally on the surface of the intestine and gently squeezing along the outer surface of the intestine to remove mucus from the lumen; cutting the intestine longitudinally and cutting laterally into approximately 0.5 cm pieces. PPs need to be removed with scissors at the small intestine. No PPs are present on the colon and do not need to be removed.
(5) Placing the intestinal tissue sections into a 50 mL pointed-bottom centrifuge tube and adding therein 40 mL of 4°C CMF solution. Inverting the tube up and down several times until the tissue is precipated, and pouring off the supernatant. Repeating three times until supernatant is relatively clear.
(6) Placing the tissue in a 50 mL conical flask containing 20 mL of CMF/FBS/DTE solution, sealing with a sealing film and then placing in a shaker and incubated at 37°C, 220 rpm for 20 minutes.
(7) Transferring the solution and tissue to a 50 mL pointed-bottom centrifuge tube and vortex shaking at maximum intensity for 15 s. Upon settlement of the tissue, a cell sieve being used for filtration and the supernatant being transferred to another 50 mL pointed-bottom tube.
(8) Adding 20 mL of CMF/FBS/DTE solution to the intestinal tissue block and repeating step 7; combining the supernatant from both operations; removing any debris formed; keeping the supernatant on ice.
(9) Placing the tissue sections back into a 50 mL conical flask, repeating steps 6 to 8, and combining the supernatants. Centrifuging at 400 x g for 5 minutes at 4°C. Resuspending the precipitate with 5 mL of RPMI 1640 pre-chilled to 4°C and storing on ice.
(10) Centrifuging at 400 × g for 5 minutes at 4°C. Resuspending cells with 8 mL of 44% Percoll at room temperature.
(11) Preparing several 17 × 100 mm tubes, rinsing these tubes sequentially with FBS, and discarding the FBS.
(12) Adding 5 mL of 67% Percoll to each tube and slowly adding 8 mL of 44% Percoll/cell suspension (step 13). Centrifuging at 4°C, 600 × g for 30 minutes.
(13) Taking half of the upper layer of the gradient until it is approximately 2 cm from the phase interface (cell layer). Collecting the phase interface and diluting the cells with RPMI 1640 at 4°C, using a ratio of 1:3. Centrifuging at 400 × g for 5 minutes at 4°C to allow for clumping of the precipitate.
(14) Counting live cells with Typan blue.
(15) After Incubation of the antibody, performing flow cytometry analysis.

### 2.2.7 Peritonitis modeling and inflammatory cell collection

(1) Weighing an appropriate amount of thioglycolate, dissolving it in ddH₂ O, formulating the concentration to 3% (w/v), sterilizing and ready to use.
(2) Injecting 1 mL of thioglycolate solution to the abdominal cavity of the mice.
(3) After 48 h, anesthetizing the mice and cutting a small incision in the lower abdomen to expose the muscle without damage to the abdominal cavity.
(4) Injecting slowly 5 mL of pre-chilled PBS into the abdominal cavity using a 5 mL syringe.
(5) Removing the syringe and gently rubbing the abdominal cavity to cleanse as much as possible.
(6) Laying the mouse on its side and inserting a 5 mL syringe. Pulling out the syringe slowly and recovering as much PBS as possible.
(7) Combining the recovered PBS containing monocytes/macrophages and centrifuging at 2000 rpm for 5 minutes at 4°C.
(8) Discarding the supernatant, washing and then resuspending with an appropriate amount of PBS.
(9) After staining with Typan blue, reading the cell density on a cell counter and calculating the number of cells.

### 2.2.8 Determination of monocyte adhesion to endothelial cells

(1) Growing HUVEC in a six-well plate until confluence, and then induced with 10 ng/ml of TNF-α for 6 h.
(2) Incubating HUVEC with or without Annexin A5 in DMEM containing 10% fetal bovine serum in a cell incubator for 30 min.
(3) Marking HUVEC with Hoechst (Invitrogen) to make adhesion one hour before THP-1 cells marked with CellTracker Green CMFDA were added. Then, removing unadhered cells by washing 3 times with PBS.
(4) Measuring fluorescence under a fluorescence microscope at excitation and emission wavelengths of 496 nm and 516 nm.
(5) Photographing 6 random microscopic fields (40×) in total, and directly counting the number of the adherent cells.

### 2.2.9 Cell adoptive experiment

The flowchart of the operation of the cell adoptive experiment is shown in Figure 3.
(1) EGFP gene mice being subjected to peritonitis model according to the above method, and collecting peritoneal fluid 2 days later to isolate leukocytes.
(2) Cell counting, injecting each mouse in the modeling experimental group with about 10⁵ cell counts.
(3) Dissecting the mice in the experimental group at appropriate time points for subsequent experiments.

### 2.3 Experimental results and analysis

### 2.3.1 The overall status of mice in the Annexin A5 treatment group was improved

After 12 h of enema, the mice began to have diarrhea with yellow loose stools and/or the perianal body hair being stained with loose stools; on day 2, the hair was dull and lusterless, mental lethargy, laziness, fear of food, diarrhea, body weight began to decline, most mice showed mucus stool, and some mice showed mucus and blood stool; on day 3, the inflammation in further progress, body weight continued to decline, activity and abrosia continued to decrease, and mortality rate increased rapidly; on day 4 and day 5, the body weight basically stopped decreasing, and the degree of disease did not improve significantly.

After 12 h of TNBS enema, Annexin A5 mice had significantly less diarrhea than the Model group, and also showed a dose effect within the group, with the diarrhea of the Annexin A5 0.10 mg/kg group being less serious than the 0.05 mg/kg group; on day 2, mental lethargy and slight weight loss occurred, but activity and abrosia were significantly better than the Model group; on day 3, Annexin A5 mice started to gain body weight, and their mental status, activity and abrosia obviously started to improve; on day 4 and day 5, the disease status of Annexin A5 group mice further improved.

In conclusion, Annexin A5 mice showed significantly better mental status, activity and abrosia than other groups of mice during TNBS colitis modeling.

### 2.3.2 Annexin A5 treatment alleviates colitis

Sulfasalazin (SASP) is a medication for the treatment of UC and CD. SASP was used as a positive control. The mice were executed on day 5 after TNBS-induced colitis. The colon sites of each group mice were taken for direct observation. The mice in the Model group (TNBS modeling with saline injection only) showed different degrees of hyperemia and edema, erosion and other pathological features, and the formation of small superficial ulcers, with the most obvious lesions in the lower part of the colon and progressing upward. In contrast, the colonic appearance of Annexin A5-administered mice tended to be normal, with formed feces, and normal color, and no significant macroscopic features of inflammation (Figure 4A); there was also a significant recovery in the length of the colon in the Annexin A5 group, and there was a significant difference (P< 0.05) (Figure 4B). In terms of the overall morphology of the colon, intravenous Annexin A5 effectively prevented the development and progression of colitis, especially at a dose of 0.10 mg/kg, which was superior to 150 mg/kg SASP. Figure 4 shows a schematic representation of the morphology and length measurements of the colon in the TNBS-induced colitis model of the present invention. FIG. 4A: Colonic morphology observation; FIG. 4B: Colon length measurement results statistics. sham control, normal mouse colon; Model, TNBS enema only; SASP, SASP treatment group, as positive control; Annexin A5 0.05 mg/kg, administered at 0.05 mg/kg dose; Annexin A5 0.10 mg/kg, administered at 0.10 mg/kg dose, and data statistics are mean ± SEM.

### 2.3.3 Decrease of DAI score in Annexin A5 treated mice

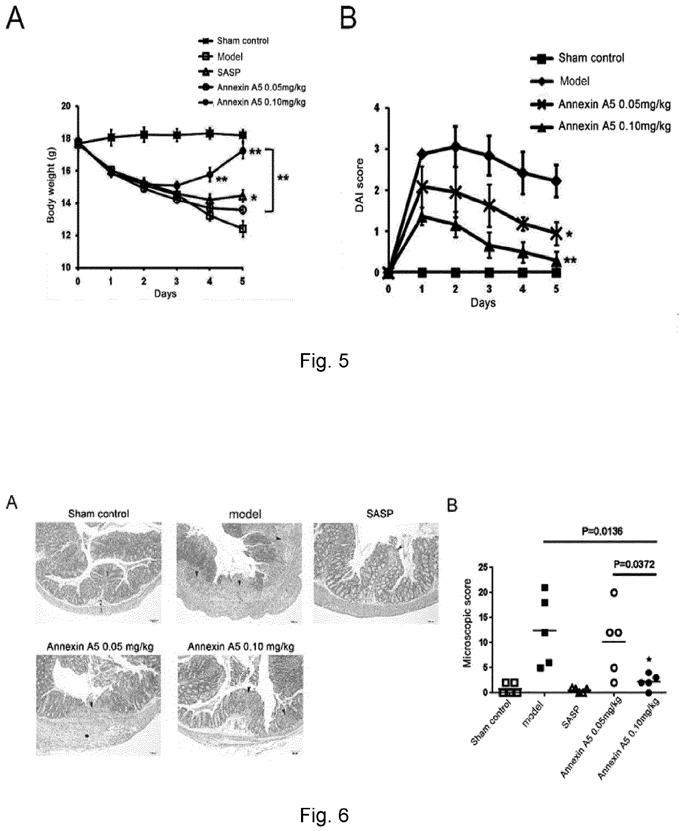

TNBS-induced colitis was usually characterized by a significant decrease in body weight. The body weight of mice decreased sharply after TNBS modeling (more than 15% after 2 days). The body weight of mice in the Model group continued to decrease, to a minimum on day 3, with little change on day 4 and day 5. The Annexin A5 0.10 mg/kg group exhibited a significantly smaller body weight decrease than the control group. The Annexin A5 0.10 mg/kg group showed significant weight recovery on day 4 after TNBS induction (P<0.01), and basically returned to normal on day 5. The Annexin A5 0.05 mg/kg group also exhibited significant weight recovery on day 5 (P< 0.05) (Figure 5A). Annexin A5 was able to restore body weight in mice.

Throughout the experiment, fresh feces of mice were measured for occult blood, which was positive or strong positive in the Model group, with occasional macroscopic blood in the flesh, and improved on day 5. The scoring was based on the above-mentioned DAI indicators, and the scoring results showed that the mice of the Annexin A5-administered group scored significantly lower than the control group (Figure 5B), indicating a decrease in disease activity.

Figure 5 shows the trend of body weight change and DAI score change over time in mice after modeling of the present invention; Figure 5A: Trend of body weight change over time in each group; the mice were weighed every morning after modeling; Figure 5B: DAI score change over time in each group. The DAI score was calculated according to the DAI calculation formula, based on daily body weight change, fecal hardness and occult blood and the data were calculated as mean ± SEM.

### 2.3.4 Reduction of pathological tissue inflammation in Annexin A5 treatment group

In normal mice, the colonic glandular ducts were regular, the intestinal wall was normal, the distribution of Goblet cells was continuous, lymphoid follicles were occasionally seen in the submucosa, a few lymphocytes were found in the lamina propria, and monocytes were almost not seen (Figure 6A). After TNBS modeling, the colonic wall of the mice in the Model group was generally thickened, the glandular ducts were misaligned or damaged, and a lot of inflammatory cell infiltration; some mice had edema in the submucosa of the colon, and in severe cases, there were multiple hemorrhages (Figure 6A).

After treatment with AnnexinA5 0.10 mg/kg, the colonic intestinal wall thickness tended to be normal, mucosal damage was barely visible, and inflammatory cell infiltration (purple areas) was similar to that of the Sham control group.

The statistical results of the pathological tissue scores of each experimental group are shown in Figure 6 B. The Annexin A5 0.10 mg/kg group was significantly different from the Model group (P< 0.05). Figure 6 shows the results of H&E staining of pathological tissue sections and pathological tissue scores after TNBS-induced colitis of the present invention.

Figure 6A shows the results of H&E staining of pathological tissue sections of the present invention; Figure 6B shows the pathological tissue scores of each group of the present invention. The degree of intestinal wall damage, lesions and recessus damage were observed and the score was calculated according to the histopathological scoring formula. The arrows show the locations of intestinal mucosa and recessus damage.

### 2.3.5 Down-regulation of inflammation-related cytokine levels in model mice

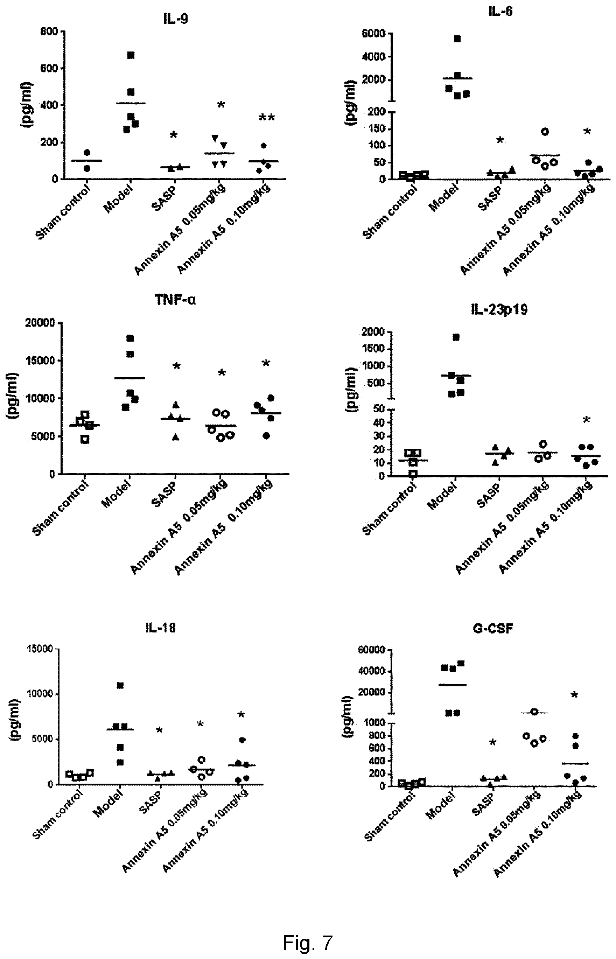

Significant increase in circulating inflammatory factors in plasma are usually associated with the severity of colitis. During the development of inflammatory bowel disease, for example, IL-23 promotes the differentiation of immune cells to produce large amount of inflammatory substance, which leads to prolonged inflammatory bowel disease. Annexin A5 significantly reduced the production of inflammatory cytokines, such as TNF-α, IL-6, IL-18, etc. Figure 7 shows the serum cytokine level assay for each group of the present invention.

Blood was collected from the eyeballs on day 5 after modeling and left to stand at room temperature for 2 h. Blood was centrifuged at 4°C for 30 min at 12000 rpm, and the upper layer serum was carefully moved into a new EP tube and labeled. Placed at -80°C for storage. The levels of chemokines and cytokines in the serum were measured using suspension microarrays as set forth in the experimental method for statistical analysis.

### 2.3.6 Reduced inflammatory cell infiltration in the colonic site of the model mice

The level of cytokines is closely related to the infiltration of inflammatory cells. When colitis occurs, a large number of neutrophils infiltrate into the intestinal lumen, and excessive aggregation of neutrophils leads to an inflammatory response and tissue damage, and there is also a positive correlation between their number and the severity of IBD. Figure 7 shows the serum cytokine levels measured in each group of the present invention. Inflammatory cell infiltration occurs in the early stage of TNBS-induced colitis. The degree of early leukocyte infiltration can reflect the severity of inflammation in colitis. Therefore, the present invention analyzes the infiltration of inflammatory cells at the colonic site. CD11b is a typical marker of neutrophils, macrophages and monocytes, and therefore CD11b was chosen as the main cellular marker.

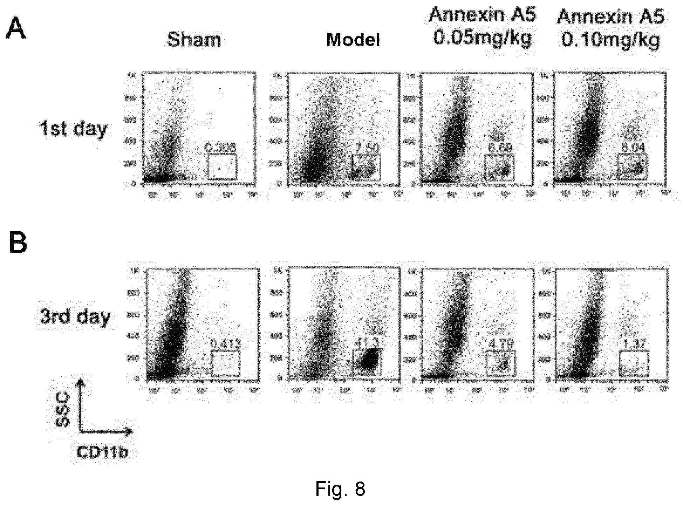

Colonic mucosal leukocytes were collected by routine procedure. Cells were enclosed with 2.4G2 hybridoma supernatant and then stained with PE-conjugated rat anti-mouse CD11b. Detection was performed on flow cytometry. The differences in colonic mucosal leukocytes between different groups were analyzed on day 1 and day 3 after TNBS induction of colitis. A small number of inflammatory cells were detected in all groups at day 1, but the number of CD11b-positive cells did not differ significantly between different groups (Figures 2-5A); however, on day 3 after TNBS induction, the percentage of CD11b-positive cell population decreased, indicating that Annexin A5 inhibited the infiltration of inflammatory cells. Figure 8 shows a schematic representation of the flow cytometry analysis of colonic mucosal leukocytes of the present invention.

Leukocytes in the colonic mucosa of each group were collected by the above method on day 1 and day 3, stained and labeled with CD11b-APC, and subjected to flow analysis. A: Leukocytes in the colonic mucosa on day 1 after induction by TNBS; B. Leukocytes in the colonic mucosa on day 3after induction by TNBS.

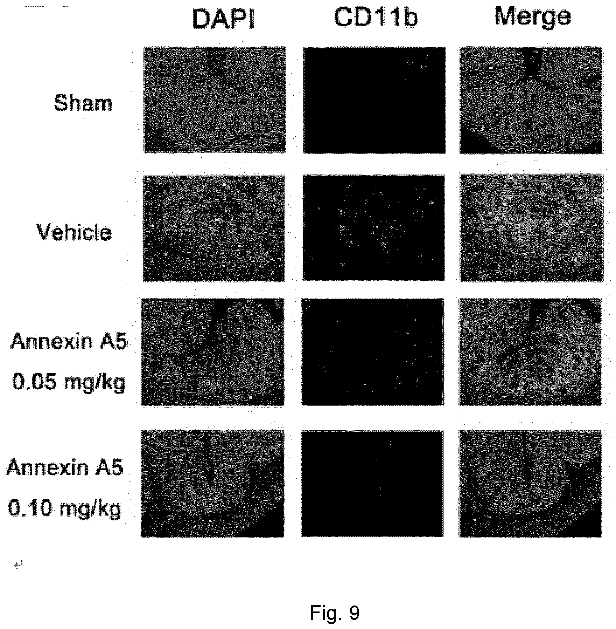

Next, the infiltration of immune cells was observed by in situ tissue observation. Modeled mice were pretreated by intravenous injection of Annexin A5 0.05 mg/kg or 0.10 mg/kg, and 20 min later the mice were executed and the colonic tissues were cut and frozen for sectioning. Compared with the Model group, it was observed by immunofluorescence that the red fluorescence of CD11b-marked inflammatory cells was almost not visible in the Annexin A5 group, indicating that there was almost no inflammatory cell infiltration in the colonic mucosa. Figure 9 shows the immunofluorescence colonic inflammatory tissue leukocytes of the present invention.

The colonic inflammation site was cut after 3 days of TNBS enema, sectioned and stained for observation. Blue is the nucleus and red fluorescence is the marking CD11b, indicating the infiltrating inflammatory cells.

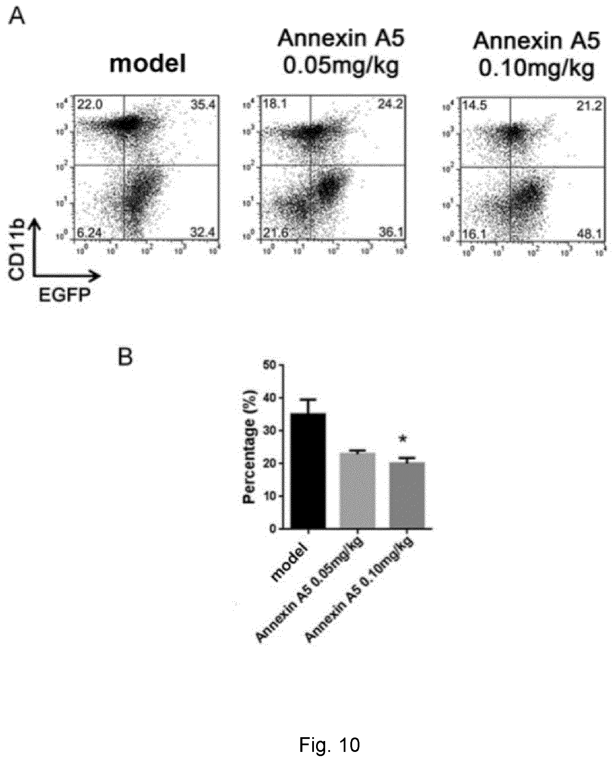

In order to observe the infiltration of inflammatory cells in mice more clearly, an EGFP+ inflammatory cell adoptive experiment was performed. Colonic lymphocytes were extracted and marked with CD11b for analysis 3 days after intravenous injection of EGFP+ cells. Annexin A5 0.10 mg/kg significantly reduced the proportion of CD11b+ EGFP+ cells compared to the Model group, indicating that Annexin A5 impeded the development of inflammation by inhibiting the infiltration of inflammatory cells at the site of inflammation. Figure 10 shows a schematic representation of the infiltration of EGFP+ inflammatory cells at the site of colonic inflammation of the present invention.

The EGFP mice were injected intraperitoneally with 3% thioglycolate solution, and peritoneal inflammatory cells were collected 2 days later. The TNBS colitis mice were pretreated with AnnexinA5, after which EGFP + inflammatory cells were injected intravenously into the model mice, and colonic mucosal leukocytes were collected 3 days later. A: Flow analysis of leukocytes in colonic tissue after injection of EGFP + inflammatory cells, with the upper right being the population of adoptive EGFP-marked inflammatory cells recruited to the colon after Annexin A5 administration; B: Flow analysis results statistical analysis, * P < 0.05.

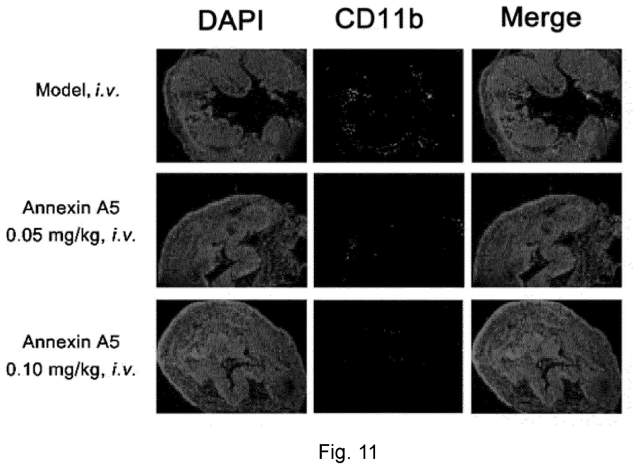

Figure 11 shows the infiltration of the adoptive EGFP + inflammatory cells in mouse colon tissue; the adoptive experiment described above was also performed to observe inflammatory cell infiltration in the in situ tissue, and the site of TNBS-induced colitis inflammation was cut after 3 days. The EGFP green fluorescence was barely visible in the Annexin A5 administration group, indicating that Annexin A5 inhibited the infiltration of the adoptive inflammatory cells in the colon tissue.

Annexin A5 is the model group pretreated with intravenous administration. Model is the model group, pretreated with saline injection only. Blue is the nucleus and green fluorescence is the adoptive EGFP+ inflammatory cells in the colon tissue.

### 2.3.7 Annexin A5 binding to endothelial prevents inflammatory cell adhesion

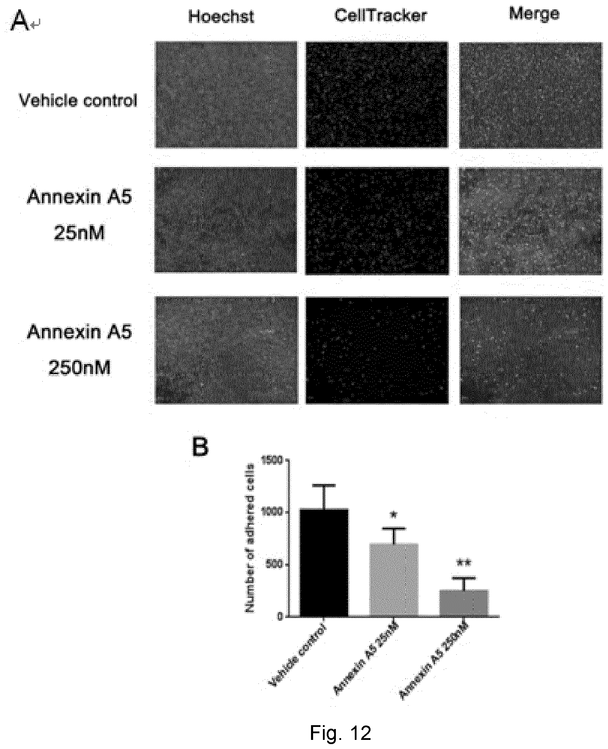

Infiltration of inflammatory cells is associated with early intercellular adhesion. To investigate the reason why Annexin A5 prevents inflammatory cell infiltration, an experiment of In vitro THP-1 adhesion to endothelial cells was performed. The results showed that Annexin A5 reduced the adhesion of THP-1 cells to HUVEC cells, a high dose of 250 nM Annexin A5 exhibited better inhibiting effect than 25 nM, and both doses were significantly different compared to the control group. Figure 12 is a schematic representation of the inhibiting effect of Annexin A5 on the adhesion of THP-1 to HUVEC of the present invention.

A: Observation of adhesion of THP-1 to HUVEC, blue fluorescence is HUVEC, green indicates THP-1 adhered to HUVEC; B: Statistical analysis of the number of adherent cells, 6 fields of view were selected for statistical analysis, * P<0.05, **P<0.01.

### 2.4 Summary and Discussion

An investigation of the disease characterization of TNBS-induced colitis treated by Annexin A5 was performed. The administration of Annexin A5 resulted in better mental status, slowed weight loss and achieved weight regain, and alleviated colon pathological features, i.e., exhibited good therapeutic effects in the aspects of physiopathological state and tissue level. Annexin A5 exhibited the effects of significantly inhibiting adhesion of inflammatory cells to the capilary endothelial cells in the model mice colonic mucosa and infiltration on the tissue, and downregulating the level of inflammatory cytokines, thereby alleviating TNBS-induced colitis.

### Example 3

PS is found in the interior of normal cell membranes, and PS on cell surface is flipped when apoptosis occurs, and can be a recognition marker for macrophages. However, many studies have also detected PS externalization in normal cells. PS exposed on most of B cells in vivo functions in receptor-mediated signaling events, does not reflect early apoptosis, and Annexin A5 can bind tumor vascular surface where PS is exposed. Therefore, PS externalization is a normal physiological phenomenon in many cells. It seems possible that capillary endothelial cells in the gastrointestinal tract undergo PS externalization in order to constantly cope with the stress of the intestinal environment, which is acidic, and the intestinal villi are in a state of constant nutrient absorption and transportation and clean up of food residues. In turn, PS externalization may mediate the adhesion of monocytes to endothelial cells and play a role in occurrence of inflammation.

In contrast, the present invention observed in this part that PS is exposed on the outer surface of colonic capillaries. Based on the high binding ability of Annexin A5 to PS, it is reasonable to speculate that Annexin A5 is enriched in colonic tissue by binding to PS. To verify this possibility, the present invention designed and purified Annexin A5mutants without PS binding ability and used the same to treat TNBS model mice, and a series of indicators were observed to determine the therapeutic effect.

### 3.1.1 Animals

C57BL/6 mice 16-18 g were obtained from the Model Animal Research Center of Nanjing University; BABL/C nude mice were obtained from Changzhou Cavens; EGFP gene mice were raised in our laboratory.

### 3.1.2 Reagents

T4 ligase and FastDigest series of restriction endonucleases were obtained from Thermo, US; plasmid extraction kit and DNA gel recovery kit were obtained from CW Reagent; BCA kit was obtained from Shanghai Beyotime; Toxin Eraser TM endotoxin removal kit was obtained from Nanjing GenScript; DL 2000 DNA Marker was obtained from Beijing Transgen; tryptone and yeast extract were obtained from Oxoid, UK; IPTG was obtained from Shanghai Biotech; other analytical reagents such as anhydrous ethanol and methanol were obtained from Nanjing Chemical Reagent Factory; primers and gene synthesis and sequencing were done by Nanjing GenScript; HEPES; DTE; bromophenol blue; trypan blue; Ni-IDA Sepharose; Superdex 200 16/600, DEAE Sepharose FFF, G50 were obtained from GE; etoposide was obtained from Nanjing KeyGen BioTECH; fetal bovine serum (FBS) and DMEM culture medium were obtained from Gibco-BRL; penicillin and streptomycin were obtained from WISENT; RPMI 1640 was obtained from Thermo; propidium iodide (PI).

### 3.1.3 Instruments and consumables

Discovery Studio was developed from Accelrys, Canada; small animal live imager was obtained from Caliper Life Sciences, US; fluorescence microscope was obtained from Carl Zeiss AG, Germany, model Zeiss AX10; automatic frozen section machine CM1950 obtained from Leica; flow cytometer obtained from BD Calibur; microplate shaker was obtained from Jiangsu Kylin-Bell; micro-thermophoresis instrument (MST) was obtained from Nano Temper, Germany; hydrophilic capillary was obtained from Nano Temper, model K003 Monolith TM NT.115; benchtop centrifuge FRESCO was obtained from Thermo, US; circular dichroism chromatograph Chirascan was obtained from Applied Photophysics, UK; autoclave was obtained from Nanjing EPED; automatic cell counter was obtained from invitrogen; pH meter was obtained from Mettler Toledo, Switzerland; high-speed centrifuge Avanti J-26S XP was obtained from Beckman, Germany; 30 kDa ultrafiltration tubes were obtained from Millipore; 100 mm cell sieve; ophthalmic scissors and gavage needle were obtained from Xinhua Medical Devices; scale; forceps; iron stand; syringe; 50 mL conical centrifuge tube; 50 mL conical flask.

### 3.1. 4 Strains and solutions

1. Prokaryotic expression vector pET-28a, E. coli clone strain Top10, and E. coli expression bacterium BL 21 (DE3) were stored in our laboratory.
2. Solutions
   (1) LB liquid culture medium: 10 g NaCl, 10 g peptone and 5 g yeast were dissolved in about 800 mL ddH₂ O and the volume was fixed to 1 L, autoclaved and stored at room temperature.
   (2) LB solid culture medium: the LB liquid culture medium was added with 1.5 % (w/v) agar, autoclaved, cooled until just not hot, poured on plate, cooled and solidified, ready to use.
   (3) 10× SDS-PAGE electrophoresis buffer: 188 g glycine, 30.2 g Tris and 150 g were dissolved in 800 mL ddH₂ O, the volume was fixed to 1 L, stored at room temperature, diluted to 1× when used.
   (4) 5 × SDS-PAGE loading buffer: 250 mM Tris-HCl (pH 6.8), 10 % (w/v) SDS, 0.5 % (w/v) BPB (bromophenol blue), and 50 % (w/v) glycerol. 5 % mercaptoethanol was added before use.
   (5) Coomassie staining solution: 1 g R-250 was dissolved in 250 mL isopropanol, 100 mL glacial acetic acid and 650 mL ddH₂ O, and mixed thoroughly.
   (6) Decolorization solution: 50 mL ethanol, 100 mL acetic acid and 850 mL ddH₂ O, mixed until uniform.
   (7) 50 mM Tris buffer (lysis buffer): 6.06 g Tris was dissolved in 800 mL ddH₂ O, the pH was adjusted to 8.0, the volume was fixed to 1 L, and stored at room temperature.
   (8) Elution buffer I: 50 mM Tris-HCl, pH 8.0, 150 mM NaCl.
   (9) Elution buffer II: 50 mM Tris-HCl, pH 8.0, 170 mM NaCl.
   (10) Hepes-NaCl buffer: 10 mM Hepes, 140 mM NaCl, pH 7.4.
   (11) Ni binding buffer: Hepes-NaCl buffer was added with imidazole having a final concentration of50 mM.
   (12) Ni wash buffer: 1 M imidazole and Hepes-NaCl buffer were mixed proportionally to make the concentration of imidazole in the solution be 50 - 100 mM.
   (13) Ni elution buffer: 1 M imidazole and Hepes-NaCl buffer were mixed in a certain ratio to make the concentration of imidazole in the solution be 250 - 300 mM.
   (14) Binding buffer: Hepes-NaCl buffer containing 2.5 mM CaCl₂.
   (15) Trypsin digest: 0.5 g crystalline trypsin and 0.2 g EDTA were added to PBS that was free of Ca2+ and Mg2+, the volume was fixed to 1 L, stirred until even with a magnetic stirrer, dissolved completely, filtered and sterilized by a 0.22 µm filter membrane, split charged, and stored at -20°C, and generally a 5× master batch was formulated.

### 3.2 Experimental method

### 3.2.1 Small animal live imaging

(1) Tail vein injection of mice, the observation method being selected according to the labeled fluorescence wavelength. Direct imaging can be done when RFP is used to observe in vivo transfer.
(2) After anesthesia, placing the mice in the imaging dark box platform, adjusting the field of view, and turning on the illumination to take a background picture.
(3) Turning off the illumination lamp automatically, taking a picture of the light emitted from the mice body under the condition of no light source.
(4) Combining the two pictures to complete the imaging operation.
(5) Using an image processing software for small animal live imaging to adjust individual images under the same scale.

### 3.2.2 Small animal imaging shows Annexin A5-TagRFP distribution in mice

(1) Injecting purified Annexin A5-TagRFP (2 mg/ kg) into nude mice through tail vein.
(2) After 20 min, displaying NIR fluorescence imaging by an IVIS Lumina XR system with a TagRFP excitation (570 nm) and emission (672 nm) filter set.
(3) Measuring the in vitro fluorescence intensity of each tissue by dissecting the mice.

### 3.2.3 Immunofluorescence detection of Annexin A5-EGFP /PS antibody distribution in mouse colon tissue

(1) Intravenous injecting PS antibody/Annexin A5-EGFP into normal mice using a 1 mL syringe
(2) Dissecting the mice 20 min later, cutting colonic and ileal tissues for frozen sectioning and drying from light.
(3) Several minutes after staining by adding dropwise of DAPI staining solution, placing under a fluorescent microscope to observe and analyze the results.

Capillaries were marked by MECA-32 antibody, externalized PS was marked by PS, and cell nuclei was stained to blue by DAPI.

### 3.2.4 PCR amplification

The PCR system was used for gene amplification, and the amplification procedure and system are shown in Table 5 below, and the PCR amplification procedure is shown in Table 6.

**Table 5**

| Components | Volume/L |
|---|---|
| 5×PrimeSTAR Buffer | 10 |
| dNTP mixture master batch | 4 |
| Primer F 1 | 1 |
| Primer R 1 | 1 |
| PrimeSTAR HS DNA Polymerase | 0.5 |
| Template | 150 ng |
| ddH₂O | Replenish to 50 |

**Table 6**

| | | |
|---|---|---|
| 98°C | 10 s | 25 cycles |
| 60°C | 15s | |
| 72°C | 1 min/kb | |
| 72°C | 7 min | |
| 4°C | **∞** | |

3.2.5 Restriction endonuclease digestion and T4-DNA ligase enzyme-linked reaction are shown in Table 7, the enzyme-linked reaction system is shown in Table 8.

**Table 7**

| Components | Volume/µL |
|---|---|
| 10 × FastDigest Green Buffer | 3 |
| Enzyme 1 | 1.5 |
| Enzyme 2 | 1.5 |
| DNA | 0.5 µg |
| ddH₂O | Replenish to 30 |
| The system may be adjusted proportionally if required, 37°C water bath sectioning reaction for 2 hours | |

**Table 8**

| Components | Volume/µL |
|---|---|
| Digested and recovered DNA fragment | 5×plasmid mole number |
| Plasmid | 50 ng |
| T4 DNA ligase | 1 |
| 10×T4 DNA ligase buffer | 2 |
| ddH₂O | Replenish to 20 |
| 16°C ligation reaction for 2 hours | |

### 3.2.6 DNA electrophoresis and DNA gel extraction

Agarose was weighed at 1% by mass/volume ratio (g/mL), dissolved by microwave heating, and when the temperature dropped to just not hot, ethidium bromide (EB) was added to a final concentration of 0.5 g/mL, poured into a gel plate inserted therein with the comb until cooled and ready to use, and the target DNA fragments were separated by electrophoresis at 150 V after spotting.

DNA gel block was cut and placed in a 1.5 mL centrifuge tube, add therein with 500 µL of binding solution, and left in a water bath at 60°C, shaking it continuously until the gel was completely melted.

The above solution was transferred to the adsorption column and allowed to stand at room temperature for 1 min, and centrifuged at 6000 rpm for 1 min. The waste liquid was discarded.

500 µL wash solution was added to the column, centrifuged at 12000 rpm for 1 min. The waste solution was discarded. This was repeated once.

The wash solution was completely removed by centrifugation at 12000 rpm for 1 min and the adsorption column was placed in a new 1.5 mL centrifuge tube.

30 µL of elution buffer was added to the center of the membrane, left for 2 min at 65°C., and then centrifuged at 12000 rpm for 1 min to collect the pass-through liquid. This step was repeated once to increase the recovery rate. The DNA-containing solution was stored at 20°C and ready to use.

### 3.2.7 Preparation and transformation of competent E. coli

(1) Competent bacteria preparation: 30 µL of overnight Top 10 bacterial solution was received in 3 mL LB, no antibiotics were added.
(2) Incubated at 37°C for 2 h, divided into two 1.5 mL centrifuge tubes and centrifuged at 4°C, 5000 rpm for 5 min. The supernatant was discarded.500 µL of pre-cooled sterile CaCl₂ (0.1 M) was added, and the precipitate was dispersed by blowing gently.
(3) Placed on ice for 30 min and then centrifuged at 5000 rpm for 5 min at 4°C.The supernatant was discarded. 100 µL of CaCl₂was added for resuspension, and then placed on ice for 2 h.
(4) Competent bacteria transformation: 100 µL of competent cells and 20 µL of ligand product or an appropriate amount of plasmid were mixed uniform, ice bath for 30 min, heat-stimulated at 42°C for 90 s, and inserted quickly into ice, let stand for 5 min
(5) 600 µL LB culture medium was added, and then vibrated for 1 h at 37°C, 220 rpm.
(6) Centrifuged at 4500 rpm for 5 min, part of the supernatant was removed, and the bacteria were resuspended.
(7) An appropriate amount of bacterial solution was taken to coat the plate (for plasmid-transformed bacteria, 100 µL of stock solution was taken to coat the plate), and incubated overnight at 37°C in the inverted position.

### 3.2.8 Protein expression and purification

### 1. Purification of Annexin A5/Annexin A5m

(1) E. coli BL 21 transformed with the expression vector was grown in 1 L LB culture medium supplemented with 50 mg/L kanamycin at 37°C and then induced with 0.4 mM IPTG (1:1000 v/v) at 16°C for another 16 h before harvesting.
(2) The harvested cells were resuspended in 50 mL of lysis buffer (50 mM Tris-HCl, pH 8.0) and sonicated at 4°C. After completion of lysis, the supernatant was collected by centrifugation at 4°C, 12000 rpm for 30 min. The ultrasonic crushing parameters were: power 300 W, sonication 4 s, pause 6 s, and sonication 30 min.
(3) The chromatography column, the protein UV absorption detector and the constant flow pump were connected through a hose, and the binding buffer (50 mM Tris-HCl, pH 8.0) was pumped into the chromatography column by the constant flow pump; the packing was pre-equilibrated and the equilibration volume was about 5~10 times of the packing volume. The UV detector was adjusted to have 100% light transmission and 0 light absorption.
(4) After the molecular sieve gel column (Superdex 200 16/600) had been equilibrated, the protein suspension was added after the equilibrium liquid level was flush with the packing. After all the protein solution had been pumped into the packing, it was eluted with elution buffer I. The protein solution was collected in small amounts several times. 12% SDS-PAGE was used to detect the collected target proteins.
(5) The anion exchange column loaded with DEAE-Sepharose was equilibrated with binding buffer (50 mM Tris-HCl, pH 8.0) for about 5 column bed volumes of binding buffer.
(6) The protein sample after Superdex 200 separation was pumped into the chromatography column by a constant-flow pump, the heteroprotein was eluted by elution buffer I, and the target protein was eluted by elution buffer II.
(7) The purity of the proteins collected in each step was examined on 10% SDS-PAGE gel. BCA method was used to determine the protein concentrations.

### 2. Purification of Annexin A5-TagRFP and Annexin A5m-TagRFP

(1) The culture solution was transferred to a centrifuge tube, centrifuged at 4°C and 6000 rpm for 15 min, the bacterial cells were washed with pre-cooled Hepes-NaCl buffer, centrifuged again at 6000 rpm for 15 min, and the bacterial cells were harvested.
(2) The bacterial cells were resuspended in 40 mL Ni binding buffer, and sonicated at low temperature, with the sonication conditions being sonication for 4 s, pause 6 s and then sonication for 30 min until the bacterial solution was clear.
(3) After sonication was complete, the supernatant was collected after centrifugation at 4°C, 12000 rpm for 25 min. The supernatant was filtered by a 0.2 µm membrane for loading, and to avoid blocking the chromatographic column bed.
(4) 5 mL of Ni agarose gel medium was taken and added to the chromatographic column, and the packing material was allowed to settle naturally.
(5) The chromatography column, the UV absorption detector and the constant flow pump were connected, and the Ni-binding buffer was pumped into the chromatography column by the constant flow pump; the packing was pre-equilibrated and the equilibration liquid volume was about 5 to 10 times of the packing volume. The UV detector was adjusted to have 100% light transmission and 0 light absorption.
(6) The supernatant filtrate was passed through with the constant flow pump.
(7) The protein unbound to Ni column was eluted with washing buffer until the OD280 value dropped and stabilized near the baseline.
(8) Ni-columnelution buffer was used to elute the target protein and the eluted fraction at the maximum absorption peak was collected. 20-30 mL of elution buffer was used to elute the target protein, and the target protein was collected in sections, 5 mL for each tube, and they were tested separately. All bound target proteins should be eluted while obtaining high purity and high concentration proteins.
(9) The packing was equilibrated with 5 mL of lysis buffer followed by with 5 mL of deionized water, and this was repeated 2 times, and finally the packing was equilibrated with 5 mL of 20 % ethanol, repeated 1 time, and then 10 mL of 20 % ethanol was added to the column and stored at 4°C.
(10) A small amount of protein was added to the sample buffer and boiled, then analyzed by 12% SDS-PAGE gel electrophoresis for protein purity. BCA method was used to determine the protein concentration.
(11) Endotoxin was removed by Toxin EraserTM endotoxin removal kit. See instructions for operation details.

### 3. Endotoxin removal

Bacterial endotoxin plays a key role in the process of Gram-negative bacteremia and endotoxemia, impairing the body's immune function and triggering systemic inflammatory responses or organ failure. To prevent endotoxin-induced adverse reactions in subsequent Annexin A5 injection treatment in mice, the purified protein was subjected to endotoxin removal. The Toxin EraserTM endotoxin removal kit was used to remove the endotoxin.
(1) Sample treatment: ionic strength and pH were adjusted with endotoxin-free 3 M NaCl, 0.1 M NaOH or 0.1 M HCl. pH range 7-8 was optimal. 0.15-0.5 M NaCl resulted in high removal efficiency and low sample loss.
(2) Endotoxin removal
   (a) Resin activation: the pre-assembled column was fixed vertically on the iron stand, the flow rate controller was turned on to let the protective solution flow out naturally but not dry; 5 mL of pre-cooled regeneration buffer was added slowly along the wall, with the flow rate being adjusted to 10 drops/min. When all the regeneration buffer was used, another 5 mL of regeneration buffer was added. The above was repeated at least twice.
   (b) Resin equilibrium: After the activation was completed, 6 mL of equilibrium buffer was added along the column wall, with the flow rate being adjusted to 10 drops/min, until all the equilibrium buffer was added. The column wall should be washed with equilibration buffer at least twice.
   (c) Endotoxin removal: The flow rate controller was turned off; the sample was added along the column wall using a heatless gun tip, with the flow rate being controlled to 10 drops/min. After the effluent reached 1.5 mL, started to receive the effluent using a heatless receiver tube. After all the sample flowed out, 1.5 mL-3.0 mL of equilibrium buffer was added for elution. The eluents were collected and combined. The sample concentration was measured.
(3) Column regeneration and storage

Referring to 1) to perform column regeneration for re-use. If storage was required, the column was equilibrated with 10 mL of equilibration buffer, and then an appropriate amount of regeneration buffer containing 20% ethanol was added, and then stored at 4°C.

### 3.2.9 MST analysis of protein binding ability to PS

MST is based on the phenomenon of thermophoresis of biomolecules in capillaries, resulting in changes in the properties of biomolecules, which are manifested in changes in the fluorescence distribution of the reaction system, and the fluorescence changes reflect the affinity of biomolecular interactions [18].

The MST experiment was conducted as follows.
(1) Non-fluorescent ligand preparation: 20 µL of the highest concentration unmarked PS molecules was prepared. 16 PCR tubes were labeled. 10 µL of buffer was added to each of 15 PCR tubes. 10 µL from PCR tube #1 was transferred to PCR tube #2 and mixed thoroughly. 10 µL from PCR tube #2 was transferred to PCR tube #3 and mixed thoroughly. Repeat this step in all sample tubes. Finally, 10 µL from PCR tube #16 was taken out and discard.
(2) Fluorescent ligand preparation: The fluorescent ligand was diluted to 2 times the concentration to be tested, and 10 µL was added respectively to PCR tubes 1-16 in sequence using a clean pipette tip, starting from the low concentration PCR tube to the highest concentration PCR tube.
(3) Incubation: The reaction solutions of the above tubes were mixed thoroughly and incubated for 5 min at room temperature away from light.
(4) Sample loading: The PCR tubes were tilted so that the liquids were siphoned into the Monolith NT capillaries, and the capillaries were loaded into the tube slots of the sample tray in the order from high concentration to low concentration.
(5) MST measurement: The various parameters required for Monolith NT.115 were set, and the measurement was performed in accordance with the set conditions.

### 3.2.10 Circular dichroism detection of protein structures

The secondary structure of macromolecules can be obtained by circular dichroism spectra scan measuring biological macromolecules such as proteins, and circular dichroism spectra has been applied to various fields of protein research.

Buffer replacement: 20 mM PB, pH 7.4, was used as the buffer for the circular dichroism scan. Accordingly, G50 packing was selected.
(1) 2 g of G50 was soaked overnight in 20 mL of 20 mM PB.
(2) Column filling: the packing was degassed and filled into the chromatographic column, naturally settled, and the column was equilibrated with 5 column volumes of PB solution.
(3) Sample loading: When the liquid level in the column dropped to the packing level, the protein sample was added to the column and eluted with PB, with the flow rate as low as possible.
(4) The fractions were collected and then concentrated in an ultrafiltration tube.

The circular dichroism (CD) signal was detected on Chirascan at room temperature. Spectra were recorded in the far ultraviolet (UV) region using a 1 mm path length cuvette at a protein concentration of 7 µM in the wavelength range of 190 to 250 nm. The spectra were obtained at a scan rate of 200 nm/min, with a 1 nm bandwidth and 0.5 s integration time. Each spectrum is the average of three scans.

### 3.2.11 Cell culture and apoptosis detection

(1) A549 cells were cultured in a six-well plate in DMEM medium containing antibiotics and 10% FBS, with the 10% FBS supplemented with penicillin and streptomycin (100 U/ mL).
(2) A549 cells were treated with 25 µM etoposide for 48 h. The culture medium was discarded and the cells were washed twice with PBS. Then digested by 0.25% trypsin and the supernatant was discarded after centrifugation at 2500 rpm for 5 min at 4°C. After washed twice with PBS, the cells were resuspended in the binding buffer.
(3) Annexin A5/A5m-EGFP and PI were added to a final concentration of 1 µg/mL. The mixture was incubated for 20 min at room temperature, protected from light.
(4) Washed twice with PBS and then used for FACS analysis.

### 3.3 Experimental results and analysis

The present invention tested the therapeutic effect of Annexin A5 on TNBS-induced colitis, and the effectiveness of Annexin A5 in the treatment of colitis was evident from changes in body weight, disease activity index and inflammatory cytokine levels in mice. It has been suggested in the literature that the anti-inflammatory effect of Annexin A5 is due to its specific binding to PS. It can be seen that Annexin A5 can effectively inhibit colitis. The present invention speculates whether the therapeutic effect of Annexin A5 on colitis is based on its binding to PS. In order to verify the speculation the present invention performed mutation of the PS binding site on Annexin A5, and then purified it to obtain Annexin A5 mutant protein and further observed the therapeutic effect thereof.

### 3.3.1 Annexin A5 can be enriched to the colonic site

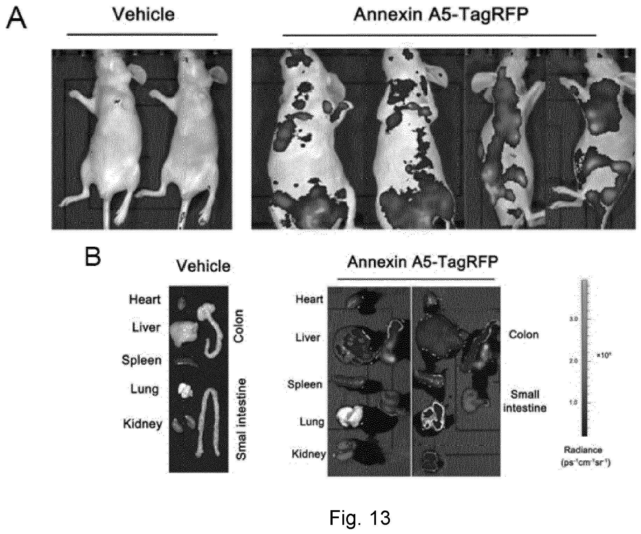

The present invention first detected the distribution of drug AnnexinA5 entering the mice body. Annexin A5-TagRFP was injected intravenously into nude mice for in vivo imaging. In vivo fluorescence imaging was performed as described in the Experimental Methods section. In vivo imaging showed that Annexin A5-TagRFP accumulated mainly at locations near the colon. After dissection, organ imaging was performed, and the off-body fluorescence of Annexin A5-TagRFP was clearly visible in the colon, with very little distribution in other organs. This showed that Annexin A5 was heavily enriched into the colon after injection into mice. Figure 13 shows the distribution of Annexin A5-TagRFP in mice tissues of the present invention.

2 mg/kg of Annexin A5-TagRFP was injected into the mice body and imaging was performed 20 min later to show the drug distribution. Figure 13A shows the in vivo fluorescence imaging of Annexin A5-TagRFP of the present invention; Figure 13B shows the organ fluorescence imaging of Annexin A5-TagRFP of the present invention. The brightness of the red fluorescence characterizes the amount of enriched protein.

In vivo Small animal imaging demonstrated the overall distribution of Annexin A5 upon entry into the body. In order to further investigate the distribution of Annexin A5 in tissues, immunofluorescence experiments were conducted. After intravenous injection of Annexin A5-EGFP, mice were dissected 20 min later, and colon segments were sectioned, placed in a dark place to dry the glass slides, stained and observed under a fluorescence microscope, and green fluorescence of Annexin A5-EGFP distributed in colon tissue was observed, and Annexin A5-EGFP was co-localized with MECA-32, but it was almost not present in the ileum (Figure 14A). The same procedure above was performed after intravenous injection of PS antibody. Immunofluorescence analysis showed that the PS antibody was also heavily distributed in the colonic capillaries, indicating the presence of exposed PS (Figure 14B). Simultaneous injection of Annexin A5-EGFP and PS antibody revealed a better co-localization of PS antibody with Annexin A5-EGFP (Figure 14C). While very few externalized PS were observed in the ileum, no enrichment of Annexin A5 was observed, either. This indicates that externalized PS occurs on the surface of colonic mucosal capillaries under normal physiological conditions and that AnnexinA5 enrichment in the colon is dependent on the presence of PS. Figure 14 shows the immunofluorescence detection of colon and ileum tissues of the present invention.

Figure 14A shows co-localization of Annexin A5-EGFP and MECA-32-labeled capillaries in the intestinal mucosa of the present invention, with blue indicating the nucleus, green fluorescence indicating AnnexinA5-EGFP protein enriched in the colon, red indicating capillaries, yellow is green-red co-localization; Figure 14B shows co-localization of PS and MECA-32-labeled capillaries in the intestinal mucosa of the present invention, with green indicating PS; Figure 14C shows co-localization of Annexin A5-EGFP and PS in the intestinal mucosa of the present invention, with green fluorescence indicating Annexin A5-EGFP, red indicating PS.

Exposure of PS is likely due to stressful conditions in the microenvironment, including metabolites, leukocytes, and hypoxic conditions. The intestine is in such an environment where cells need to respond to various stresses, so it is possible that PS in the intestine undergoes externalization, and Annexin A5 may be enriched to the colon by binding to PS exposed on the surface of these capillaries.

### 3.3.2 Construction and purification of Annexin A5 variants

To further verify the mechanism of Annexin A5 enrichment into the colon, the present invention mutated Annexin A5 so that it lost the PS binding function. An Annexin A5 variant (Annexin A5m) with eight mutant sites was constructed, such as R25A, K29S, R63S, D68A, E72Q, D144N, E228A and D303N (Figure 15).

Figure 15 shows the mutation of sites of Annexin A5 binding to PS of the present invention.

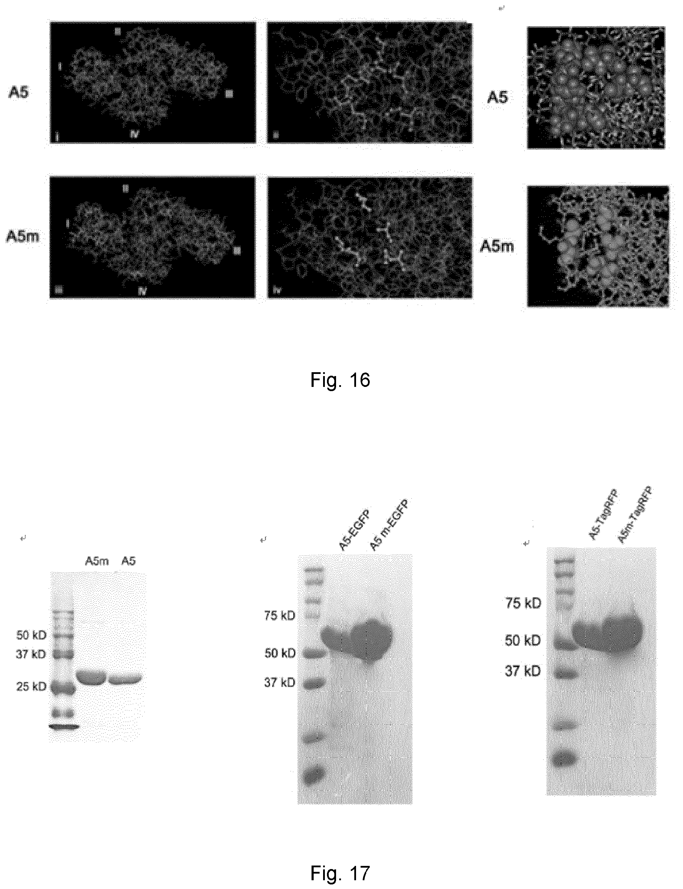

Protein molecular modeling was performed to test whether the proposed mutation could cause Annexin A5 to lose its PS-binding function. Molecular modeling showed that the amino acid residues of the five amino acids R25, K29, R63, D68 and E72 in Annexin A5 structural domain I to be used as the yellow-labeled region of the mutation site formed a pocket-like structure (Figure 16, the PS was fully chimeric with the protein molecule of Annexin A5after docking operations, and the PS fitted well into the pockets (Figure 16)). In contrast, Annexin A5m did not have a similar structure (Fig. 16), and mutations at these sites disrupted the PS-binding conformation, and Annexin A5m failed to dock with PS, indicating that the proposed mutation sites were correctly designed and that these eight site mutations was capable of disrupting Annexin A5 binding to PS. Figure 16 shows the line model of the 3D structure of Annexin A5/Annexin A5m and their docking results with PS; Figure 16 shows the 3D structure of Annexin A5 before and after mutation (yellow represents the mutated amino acid positions); Figure 16 shows the docking results of Annexin A5/Annexin A5m with PS (yellow represents the mutated amino acid sites, white represents PS).

Annexin A5m gene fragment was synthesized based on the above mutation, ligated to pET-28a plasmid, transformed into TOP 10 strain, single clones were picked for sequencing analysis, and plasmids of correctly sequenced strains were extracted. The plasmid was transformed into BL21(DE3) strain and cultured in shake flasks. After induction expression, the bacterial broth was collected for ultrasonic lysis and centrifugation.

Figure 17 shows the results of Annexin A5m-related protein purification detected by SDA-PAGE of the present invention. Annexin A5m was obtained by S200 molecular sieve for bulk protein removal, DEAE anion exchange column elution, and finally 150 mM Tris-HCl, 170 mM NaCl elution. Annexin A5m, Annexin A5m-EGFP, Annexin A5-TagRFP, Annexin A5m-TagRFP were obtained separately by carrying out experiments as described in the experimental method, and the results of SDS-PAGE gel detection showed the protein purities were over 98%.

The constructed BL21 strains were inoculated, IPTG was added at 1%o LB culture volume, induced at 16°C for 16 h, the bacterial broth was collected, lysed and then purified by following the experimental method. A. Annexin A5/Annexin A5m purification; B. Annexin A5-EGFP/ Annexin A5m-EGFP purification; C. Annexin A5 -TagRFP/ Annexin A5m-TagRFP purification.

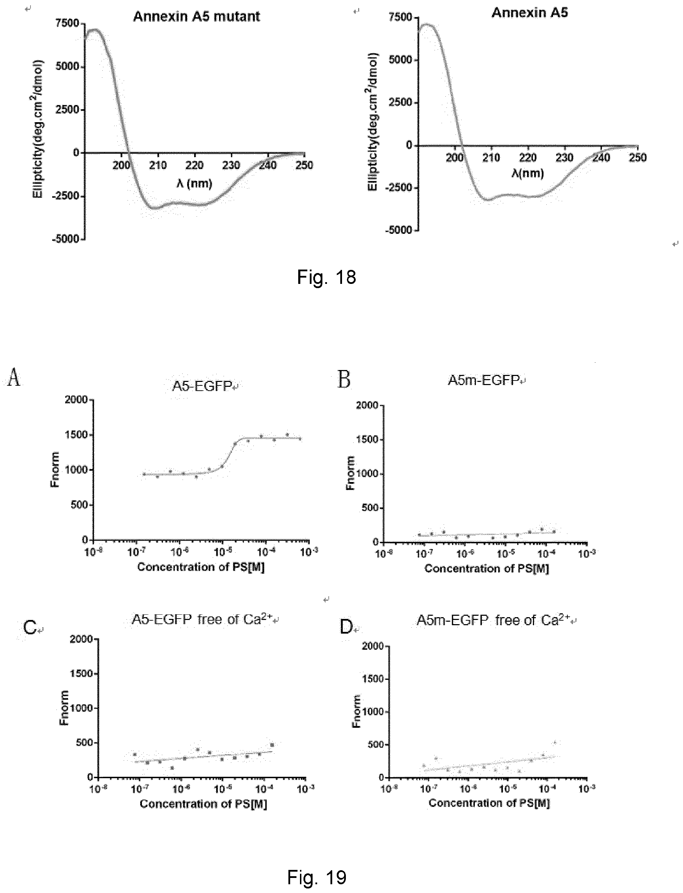

Circular dichroism (CD) spectroscopy is used to analyze conventional secondary structure features of proteins such as α-helix and β-fold. CD spectra of α-helix conformation show negative peaks at 208 nm, 222 nm, and a positive peak near 190 nm. Circular dichroism experiments were performed to detect changes in the secondary structure of the purified protein before and after the mutation. AnnexinA5 is an α-helical structure protein, the scan curves of both were identical (Figure 3-6, A, B). Based on the CD signals from both proteins, they have the same secondary structure. Figure 18 shows the secondary structures of Annexin A5m and Annexin A5 detected by the circular dichroism of the present invention.

The protein buffer was replaced with 20 mM PB solution, the protein concentration was adjusted to 7 µM, and the spectra were recorded in the far ultraviolet (UV) region at room temperature using a 1 mm path length cuvette in the wavelength range of 190 to 250 nm. The spectra were obtained at a scan rate of 200 nm/min with a 1 nm bandwidth and 0.5 s integration time. Each spectrum is the average of three scans. A. Circular dichroism scan results for Annexin A5m; B. Circular dichroism scan results for Annexin A5.

The molecular docking model only simulated the binding of Annexin A5 to PS. In order to confirm whether the mutation can cause the loss of binding ability to PS, the present invention also needs to detect the binding at multiple levels. Firstly, a highly sensitive MST was chosen for the detection at the molecular level. MST detection was performed using Annexin A5-EGFP and Annexin A5m-EGFP obtained by purification to compare the affinity of both to PS. The binding curves were plotted using Fnorm. Annexin A5-EGFP formed different detection curves due to change of its own thermophoretic motion after binding to PS. It was found that Annexin A5-EGFP and PS were analyzed and fitted into a binding curve, and the two was bound with each other, with a Kd value of 0.0138 mM.Kd value is a reflection of the protein's ability to bind to PS. However, Annexin A5m-EGFP failed to be fitted into a binding curve and with no Kd value, indicating that it did not bind to PS (Figure 3). Figure 19 shows the binding ability analysis (MST) of Annexin A5-EGFP/Annexin A5m-EGFP with PS of the present invention.

The concentration of fluorescent protein molecules was fixed, gradient dilution was performed for non-fluorescent PS, and the reaction sample without Ca²⁺ was used as a negative control. A. Annexin A5-EGFP fitting curve in the presence of Ca²⁺; B. Annexin A5m-EGFP fitting curve in the presence of Ca²⁺; C. Annexin A5-EGFP fitting curve without Ca²⁺; D. Annexin A5-EGFP fitting curve without Ca²⁺.

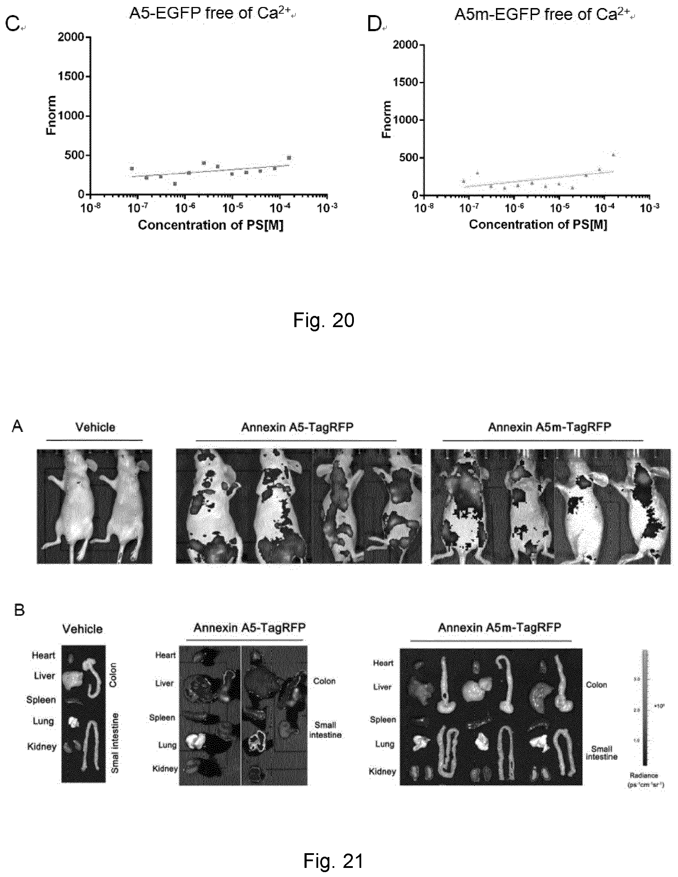

Assays at the molecular level have basically demonstrated that Annexin A5m loses its ability to bind to PS. This was followed by a binding assay at the cellular level. Apoptosis was induced in A549 cells and flow analysis was performed using Annexin A5-EGFP/A5m-EGFP co-stained with PI. Annexin A5-EGFP+ cells with a percentage above 90% normally labeled apoptotic cells; whereas Annexin A5 m-EGFP was unable to detect apoptotic cells (Figure 20). annexin A5m-EGFP did not bind to PS. Figure 20 shows a graph of Annexin A5-EGFP/Annexin A5m-EGFP of the present invention to detect A549 cells that have been induced to apoptosis.

Annexin A5m-TagRFP was injected into the tail vein, and 20 min later the mice were anesthetized and small animal imaging was performed. The red fluorescence did not show up in the colonic site, indicating that Annexin A5m could not be enriched in the colonic site, and this was confirmed by imaging of various tissues and organs.

Figure 21 shows the distribution of Annexin A5-TagRFP/Annexin A5m-TagRFP in vivo and in organs of mice of the present invention. FIG. 21A shows the distribution of Annexin A5-TagRFP/Annexin A5m-TagRFP in live mice of the present invention; FIG. 21B shows the distribution of Annexin A5-TagRFP/Annexin A5m-TagRFP in organs of the present invention. Vehicle is a control group injected with saline only. Red fluorescence intensity characterizes protein enrichment.

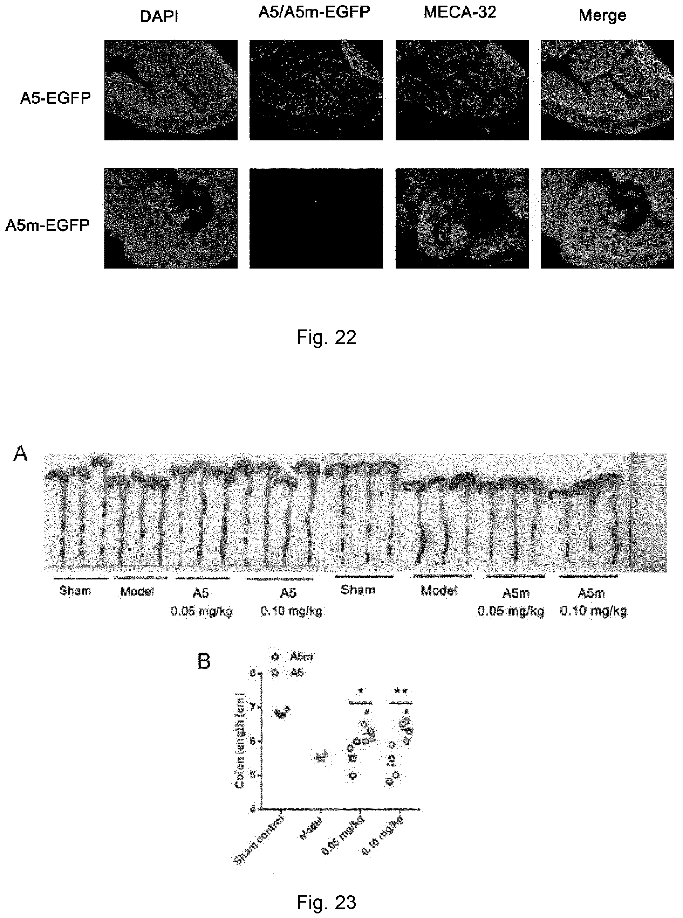

Figure 22 shows the localization of Annexin A5/A5m-EGFP in colon tissue of the present invention. As expected, Annexin A5m-EGFP could not be detected in colon tissue sections (Figure 22). Annexin A5-EGFP was able to enrich heavily into the colon, while no enrichment of Annexin A5 m-EGFP could be observed, indicating that the mutated Annexin A5 was unable to bind to the externalized PS of the colonic capillary. Annexin A5 enrichment in colon was dependent on its PS binding activity. Blue represents the nucleus, green represents AnnexinA5/A5m-EGFP enrichment in the colon, and red represents the colonic mucosal capillaries.

### 3.3.3 AnnexinA5 variants do not have efficacy in treatment of TNBS-induced colitis

The situation of Annexin A5m group mice was basically the same as that of Model group mice, and there was no difference between groups with different doses. On day 2, mental lethargy and sharp weight loss appeared; on day 3, the mental status, activity and abrosia of Annexin A5m group still did not improve significantly; on days 4 and 5, the situation of Annexin A5m mice was similar to that of the Model group. In conclusion, the mental status, activity and abrosia of Annexin A5m mice were significantly worse than Annexin A5 mice during the TNBS colitis modeling.

The measurement of the length of the mouse colon revealed that after TNBS-induced colitis, the length of the colon of the mice in the Annexin A5 group tended to be normal, while the Model group showed a significant shortening, i.e., a form of colonic lesion, and there was no significant difference between the Annexin A5m administration treatment group compared with the Model. Statistical analysis of the colon length revealed that there was no significant difference in the Annexin A5m administration group compared to the Model group and they were significantly lower than the Annexin A5 group.

Figure 23 shows the appearance and length measurement of the colon of mouse with TNBS-induced colitis of the present invention. Fig. 23A: Colonic appearance of each group mice of the present invention, Sham is normal mice, Model group is perfused with TNBS only, A5/A5m is administration group respectively. Fig. 23B: Colonic length statistics of each group of the present invention. Data are mean ± SEM, *P<0.05, ** P<0.01, Annexin A5 group vs. A5m group; #P<0.05, ##P<0.01, Annexin A5 group vs. Model group.

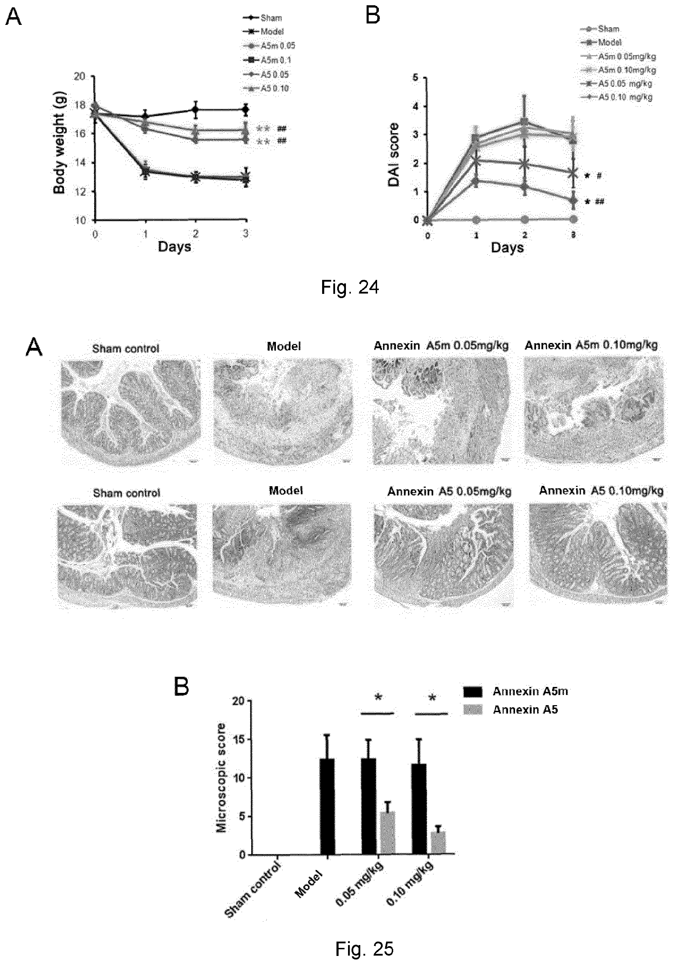

The mice in the Annexin A5m group never showed any significant regain in body weight, in line with the trend of the Model group. The DAI score showed no significant difference in disease activity between the Annexin A5m group and the Model group (Figure 24B). Figure 24 shows the trend of body weight change and DAI score of the mice of the present invention. Figure 24A shows the trend of body weight change over time for each group of the present invention; Figure 24B shows the DAI score change over time for each group of the present invention. *P<0.05, ** P<0.01, Annexin A5 group vs. A5m group; data are mean ± SEM, #P<0.05, ##P<0.01, Annexin A5 group vs. Model group. The therapeutic effect was not obvious for the AnnexinA5m group, whose intestinal wall was significantly thickened and whose intestinal mucosa and villi were severely damaged, the inflammatory cell infiltration was significantly increased, which were not significantly different from the Model group. The pathological scoring showed that the score for the AnnexinA5m group was significantly higher than that for the AnnexinA5 group.

Figure 25 shows the H&E staining and pathological score of the colon tissue of TNBS-induced colitis mice of the present invention. A: Observation of H&E-stained section of colon tissue; B: Histopathological scores of each tissue.

### 3.3.4 Annexin A5 variants do not affect the infiltration of inflammatory cells at the colonic site

To investigate the effect of Annexin A5/A5m on inflammatory cell infiltration at the site of inflammation, EGFP+ inflammatory cells were adoptively delivered into mice body, and mucosal leukocytes were collected from a colon segment of the model mice, and EGFP+ CD11b+ cells in the colon mucosa were quantified by FACS. Incubated by CD11b antibody and then sample was loaded for analysis. The proportion of EGFP+ CD11b+ cells in the Annexin A5m group reached 40% or above, and pretreatment by Annexin A5m did not reduce inflammatory cell infiltration. Annexin A5m did not prevent the recruitment and infiltration of adoptive inflammatory cells at the site of colonic inflammation.

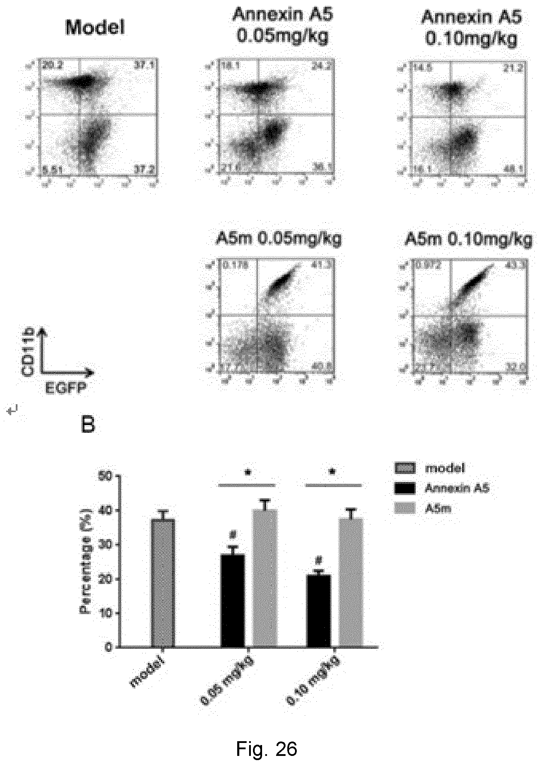

FIG. 26 is a schematic representation of the flow cytometry analysis of colonic mucosal leukocytes of the present invention. A: FACS analysis of EGFP+ CD11b + cells in colonic mucosal leukocytes, with EGFP+ inflammatory cell population on the upper right; B: Statistical analysis of the percentage of EGFP + CD11b + subpopulations in colonic mucosal leukocytes. * P < 0.05, Annexin A5 group Vs. A5m group; # P < 0.05, Annexin A5 group Vs. Model group.

### 3.4 Summary and Discussion

The present invention revealed that Annexin A5 was well enriched in the colon, and further immunofluorescence detection revealed PS eversion in capillaries within the intestinal mucosa, and Annexin A5 co-localized with the PS externalized sites, indicating that Annexin A5 was enriched in intestinal tissues by binding specifically to PS. The present invention designed to expressed Annexin A5 variant A5m, which lost PS binding ability. It was demonstrated that when Annexin A5 lost PS binding ability, Annexin A5m could not enrich into the colon and prevent inflammatory cell infiltration, and thus could not relieve intestinal inflammation. This fully indicates that the therapeutic effect of Annexin A5 is dependent on its binding to PS, which can be applied to the therapeutic strategy of targeting drug delivery based on PS externalization and it is expected to be developed as an anti-inflammatory drug applied to the treatment of IBD.

The present invention found that the level of Annexin A5 was significantly decreased in inflamed colonic mucosal compared to normal colonic mucosal. It is the first time that a correlation between colitis and AnnexinA5 was found. The literature has reported that Annexin A5 has anti-inflammatory function, so the present invention speculates that the addition of exogenous Annexin A5 may be a therapeutic strategy for colitis. When used in TNBS-induced colitis, Annexin A5 was found to be effective in treating colitis. The inhibitory effect of Annexin A5 on inflammatory cell infiltration was clearly observed by EGFP+ inflammatory cells injected into the TNBS-induced colitis mice. The drug distribution was observed by injecting Annexin A5 fluorescent protein into mice, and Annexin A5 was found to be enriched into colonic tissues. Additionally, immunofluorescence experiments revealed co-localization of Annexin A5 with externalized PS in colonic tissues, suggesting that PS exposure on colonic mucosal capillaries may provide a binding target for Annexin A5. To demonstrate this the present invention mutated Annexin A5 protein to make it unable to bind to PS, and it was found that Annexin A5m, which had lost PS binding function, was unable to be enriched into the colon, could not prevent migration and infiltration of inflammatory cells, and was unable to treat TNBS-induced colitis.

After Annexin A5 was injected into mice, it was enriched into the colonic mucosal capillaries and bound to the externalized PS of capillary endothelial cells, and PS, as a signal for inflammatory cell migration and infiltration, was blocked. Inflammatory cell adhesion and infiltration were reduced, thus preventing the development of inflammation.

Blockade of tumor necrosis factor (TNF) is commonly used as a standard clinical therapy for IBD, which suppresses inflammation by blocking signals or molecules upregulated by TNF-α. However, long-term use of TNF-α antibodies has adverse effects, including skin lesions, immune response, infection and increased risk of cancer. Vedolizumab has been approved for the treatment of severe ulcerative colitis, and it is designed to inhibit cellular infiltration by specifically antagonizing the integral protein α4β7 to block intestinal mucosal addressin. In the present inventive study, Annexin A5 enriched in colonic capillaries significantly reduces inflammatory cell infiltration into the site of TNBS-induced colitis. Targeted drug delivery (TDD) is an effective strategy for treating disease with minimal harmful side effects. The findings of the present invention suggest that PS externalization could be a potential target for therapeutic strategies of targeted drug delivery (TDD) and that Annexin A5 could be developed as a promising approach for the treatment of IBD.

The principle, main features and the advantages of the present invention have been shown and described. One skilled in the art should understand that the present invention is not limited by the above embodiments, and what is described in the above embodiments and specification is merely to illustrate the principle of the present invention, and there will be various modifications and improvements to the present invention without departing from the spirit and scope thereof. The scope of the present invention is defined by the appended claims, specification and equivalents thereof.

## Claims

1. Use of phosphatidylserine in preparation of a drug for treatment of inflammatory bowel disease, **characterized in that** for the inflammatory bowel disease, phosphatidylserine serves as a target of the drug.

2. The use of phosphatidylserine in preparation of a drug for treatment of inflammatory bowel disease according to claim 1, **characterized in that** in the inflammatory bowel disease, phosphatidylserine of vascular endothelial cells is flipped from the intracellular membrane to the extracellular membrane, and the externalized phosphatidylserine serves as a marker of the inflammatory bowel disease.

3. The use of phosphatidylserine in preparation of a drug for treatment of inflammatory bowel disease according to claim 1, **characterized in that** marked Annexin A5 is capable of being traced by phosphatidylserine in the extracellular membrane and indicating location of lesions and extent of the inflammatory bowel disease.

4. The use of phosphatidylserine in preparation of a drug for treatment of inflammatory bowel disease according to claim 1, **characterized in that** the drug is screened by using phosphatidylserine as the target for the inflammatory bowel disease to obtain proteins and compounds having a high affinity to phosphatidylserine.

5. The use of phosphatidylserine in preparation of a drug for treatment of inflammatory bowel disease according to claim 4, **characterized in that** the drug exerts anti-inflammatory effects by binding to phosphatidylserine to inhibit inflammatory cell infiltration and block recruitment of colonic immune cells.

6. The use of phosphatidylserine in preparation of a drug for treatment of inflammatory bowel disease according to claim 5, **characterized in that** Annexin A5 exerts anti-inflammatory effects by binding to phosphatidylserine.

7. A method of modifying a drug that uses phosphatidylserine as a target for inflammatory bowel disease, **characterized in that** Annexin A5 is mutated to enhance its binding ability to phosphatidylserine to improve anti-inflammatory effects thereof.
